# EUROPEAN PATENT APPLICATION

(11) **EP 3 318 340 A1**
(43) Date of publication of application: **09.05.2018**
(21) Application number: 16206444.8
(22) Date of filing: 22.12.2016
(51) Int. Cl.: B08B 9/08, B08B 9/093, C07C 41/00

(54) **METHOD FOR WASHING SEVOFLURANE STORAGE CONTAINER AND METHOD FOR STORING SEVOFLURANE**

(30) Priority: 02.11.2016 JP 2016215676; 20.12.2016 JP 2016246253
(71) Applicant: Central Glass Company, Limited, Yamaguchi 755-0001 (JP)
(72) Inventor: IWAO, Katsumi, Ube-shi, Yamaguchi 755-0001 (JP); YOSHIMURA, Takaaki, Ube-shi, Yamaguchi 755-0001 (JP); OONO, Toshihiko, Ube-shi, Yamaguchi 755-0001 (JP); AKIBA, Shinya, Kawagoe-shi, Saitama 350-1159 (JP); FUJIWARA, Masaki, Chiyoda-ku, Tokyo 101-0054 (JP)
(74) Representative: J A Kemp

(57) **Abstract**

It is an object of the present invention to provide a method for effectively washing a used "sevoflurane storage container" without using expensive sevoflurane as a washing liquid. This object is achieved by employing the washing method, comprising the steps of: creating a state in which at least sevoflurane vapor is present in the storage container (step A); bringing a "liquid containing water as a major component" into contact with the inner wall of the sevoflurane storage container in the state in which sevoflurane vapor is present in the storage container and draining the liquid outside of the storage container while the liquid remains liquid after step A (step B); and introducing a drying gas into the storage container so as to drain the liquid remaining on the inner wall of the storage container together with the drying gas outside of the storage container after step B (step C).

## Description

### TECHNICAL FIELD

The present invention relates to a method for washing a storage container for a medicine and especially fluoromethyl-1,1,1,3,3,3-hexafluoroisopropyl ether (sevoflurane) which has been widely used as inhalation anesthetic, and a method for storing sevoflurane.

### BACKGROUND ART

Fluoromethyl-1,1,1,3,3,3-hexafluoroisopropyl ether (sevoflurane) has been widely used as a safe inhalation anesthetic for use. As disclosed in Patent Literature 1 (U.S. Patent No. 4250334), sevoflurane can be synthesized by adding concentrated sulfuric acid and hydrogen fluoride to paraformaldehyde, heating the obtained reaction mixture, and adding 1,1,1,3,3,3-hexafluoroisopropyl alcohol (HFIP) dropwise to the mixture. A substance of interest (i.e., sevoflurane) can be collected together with an unreacted substance (e.g., HFIP) by collecting a gas generated in the reaction system.

Various by-products are generated in the above sevoflurane synthesis reaction. These by-products can be separated or removed together with an unreacted substance (e.g., HFIP) via, for example, the steps of bringing sevoflurane into contact with "Brønsted acid such as concentrated sulfuric acid, Lewis acid, or an acid immobilized to a resin or the like" (Patent Literature 2: JP Patent No. 2786106), bringing sevoflurane into contact with "a basic aqueous solution such as sodium hydroxide" (Patent Literature 3: JP Patent No. 4087488), and "performing distillation and/or purification of sevoflurane under the presence of a degradation inhibitor such as sodium hydrogen phosphate" (Patent Literature 4: JP Patent No. 2786108). Accordingly, high-purity sevoflurane can be obtained. It is also known that long-term storage stability of sevoflurane can be significantly improved with the addition of a small amount of water (206 ppm to 1400 ppm) to the thus purified sevoflurane (high-purity sevoflurane) (Patent Literature 5: JP Patent No. 3664648).

Meanwhile, Patent Literature 6 (JP Patent No. 3183520) discloses that a storage container made of glass, plastic, steel, or the like can be used as a sevoflurane storage container. Patent Literature 7 (JP Patent No. 3524060) discloses that particular plastic members of polyethylene naphthalate, polymethylpentene, polypropylene, polystyrene, and the like are preferably used for a storage container, and Patent Literature 8 (JP Patent No. 5801024) discloses that an aluminium container with an inert lining of lacquer or enamel can be used preferably as a storage container.

### SUMMARY OF INVENTION

### TECHNICAL PROBLEM

As sevoflurane is an ether in the form of volatile liquid, a hermetically sealed container is used as a sevoflurane storage container.

In particular, it is common to use a large-size hermetically sealed container (e.g., a container with an inner volume of 500 dm³) as a storage container in order to ship and transport sevoflurane as an "active pharmaceutical ingredient." As such large-size hermetically sealed container is very expensive, it is necessary to repeatedly use the container for a series of lots. Specifically, sevoflurane is produced as an active pharmaceutical ingredient, filled into such large-scale hermetically sealed container, and then shipped. A consumer conducts a sevoflurane consumption step (referred to as a step of evaluating quality of sevoflurane as a medicine and dispensing sevoflurane as a medicine into glass ampules and the like) and then returns a storage container containing a minute amount of sevoflurane to a manufacturer. The manufacturer fills the container with newly synthesized sevoflurane for a new lot and ships the product again.

Here, if sevoflurane is shipped as an active pharmaceutical ingredient, a hermetically sealed container is filled with sevoflurane that accounts for a large part of the active pharmaceutical ingredient, which preferably means that a gas phase (i.e., a void in the container) is filled with an inert gas (e.g., nitrogen gas). More preferably, based on the disclosure of Patent Literature 5, a small amount of water (206 to 1400 ppm) is added as a stabilizer to sevoflurane so that sevoflurane falls under conditions with improved stability. Under such strictly controlled conditions, sevoflurane is highly stable, and therefore, it is not a substance that can be easily degraded.

However, after sevoflurane is subjected to the consumption step, a small amount of sevoflurane in the liquid or gas form remains in the sevoflurane storage container and coexists with a gas phase (filled with an inert gas or air) with a relatively high volume until the sevoflurane storage container is returned to the manufacturer. After sevoflurane is left under such conditions for a long period of time, it may behave differently than sevoflurane in the usual state of "being filled into the hermetically sealed container." In particular, the present inventors have found that if sevoflurane in a liquid phase has been in contact with a large amount of drying gas for a long period of time, moisture in sevoflurane preferentially evaporates, resulting in a distinctive behavior of liquid sevoflurane, which is a gradual decrease in the moisture content (see "Reference Example 1" below).

Meanwhile, as disclosed in Patent Literature 5 and described in the Examples below, when sevoflurane is exposed to stringent conditions while its moisture content is significantly low, a portion of sevoflurane may be converted into an analog compound, which is called "polyether" (typically "polyether 1" or "polyether 2" shown below).

(CF₃)₂CHO-CH₂-O-CH(CF₃)₂ 1,1,1,3,3,3-Hexafluoro-2-[[2,2,2-trifluoro-1-(trifluoromethyl)ethoxy]methoxy]propane < Polyether 1 >

(CF₃)₂CHO-CH₂-O-CH₂-O-CH(CF₃)₂ 2,2'-[Oxybis(methyleneoxy)]bis[1,1,1,3,3,3-hexafluoropropane] < Polyether 2 >

Since sevoflurane is a medicine (anesthetic), such transformation product must not be contained in sevoflurane for the next lot, even if the content of the transformation product is very small. Note that "Official Monograph of Sevoflurane" of the Japanese Pharmacopoeia, Seventeenth Edition (JP17) states in p. 996 that the concentration based on the peak area of gas chromatography for each impurity other than sevoflurane and hexafluoroisopropyl methyl ether in a sevoflurane product must be not more than 25 ppm per compound, and the total concentration of impurities must be not more than 50 ppm. In addition, such "impurities" are mainly assumed to be by-products generated in sevoflurane synthesis and purification steps. That is, such impurities are not assumed to be compounds that are generated from sevoflurane for the previous lot in the storage container.

Further, it may take a relatively long period of time (e.g., 6 months) to return the storage container to the manufacturer due to the transportation issue after the completion of the "consumption step."

Sevoflurane is originally a highly stable compound. However, in consideration of the above circumstances, it might be impossible to prevent undesirable side reactions from taking place after the completion of the "consumption step" until the return of the container. For such reason, in order to guarantee quality of sevoflurane with certainty, when the manufacturer refills a container with sevoflurane for a new lot, it is essentially necessary to wash well the inside of the container so as to completely wash away sevoflurane filled into the container for the previous lot outside of the system.

As an aside, as a method for washing the inside of a container accommodating a liquid (such as a liquid compound or liquid composition), washing with a "liquid identical to the liquid" is often carried out. Such washing operation is usually called "rinsing," which is widely used in container washing methods especially for solution analysis. In the case of sevoflurane, as it is expected from the fact that each of the above analog compounds (polyether 1 and polyether 2) shares the basic structure with sevoflurane, the analogs have high affinity for sevoflurane. Therefore, the present inventors thought that rinsing with "purified sevoflurane" (also referred to as "rinsing with sevoflurane") is particularly preferable. Such washing method has been employed for many years in a manufacturing setting.

However, in order to carry out "rinsing with sevoflurane" to an extent that allows complete removal of impurities and ensure the removal with certainty, it is necessary to repeat "rinsing with sevoflurane" many times in principle. This inevitably causes an increase in the consumption of "sevoflurane." As "sevoflurane" is an expensive product, "rinsing with sevoflurane" leads to waste of resources, which has been an economically disadvantageous factor. That is, the development of a washing method that can replace such rinsing method has been awaited.

In general, a "method for using an organic solvent as a washing liquid" has been known as a method for washing a container. However, sevoflurane is an "inhalation anesthetic," and therefore, it must not be mixed with even a minute amount of an organic solvent. Examples of solvents having high affinity for sevoflurane and its analogs include methanol, acetone, and diethyl ether. These examples are solvents having relatively low boiling points. Such solvents might be considered to be easily removed by drying means. However, in fact, it is rather difficult to wash a sevoflurane storage container using the above solvents. Specifically, it is actually possible to remove a large portion of such an organic solvent after washing a storage container with the solvent and introducing hot air into the storage container or evacuating the storage container. If a container treated by such drying means is filled with sevoflurane for a new lot, a slight peak of the solvent might be detected in the peak of sevoflurane in a gas chromatograph (see "Comparative Example 1" below for details). If an organic solvent is detected from sevoflurane for a new lot, the relevant product will be rejected.

That is, the use of the above organic solvents as washing liquid for a "pharmaceutical product of sevoflurane" that must not contain an impurity even at the ppm level could cause burden for quality control. Thus, the use of such solvents has never been preferable.

In view of the above circumstances, an object of the present invention is to provide a novel method for washing a sevoflurane storage container.

### SOLUTION TO PROBLEM

As a result of intensive studies in order to achieve the object, the present inventors have found an excellent method for washing a used "sevoflurane storage container" with a "liquid containing water as a major component." Specifically, the present invention relates to a method for washing a used "sevoflurane storage container," the method comprising the steps of:
creating a state in which at least sevoflurane vapor is present in the storage container (step A);
bringing a liquid containing water as a major component into contact with the inner wall of the sevoflurane storage container in the state in which sevoflurane vapor is present in the storage container after step A and draining the liquid outside of the storage container while the liquid remains liquid (step B); and
introducing a drying gas into the storage container so as to drain the liquid remaining on the inner wall of the storage container together with the drying gas outside of the storage container after step B (step C).

According to the present invention, a "sevoflurane storage container" that is an object to be washed (i.e., the above used "sevoflurane storage container") is a sevoflurane storage container, which is repeatedly used for a series of lots, the storage container having been subjected to a step of consuming at least a portion of sevoflurane filled into the storage container for a previous lot (typically, substantially all or all of sevoflurane filled into the container).

The present invention is characterized by "step A" described above, that is to say, the step of creating a state in which at least sevoflurane vapor is present in the storage container. By conducting "step A," it is possible to continuously keep the inner wall of the sevoflurane storage container in contact with sevoflurane molecules, thereby making it possible to effectively conduct washing with a "liquid containing water as a major component" in step B.

Specifically, it has been attempted to allow the sevoflurane storage container (in particular, the inner wall thereof) to have affinity for sevoflurane molecules by "step A," bring a "liquid containing water as a major component" into contact with the inner wall of the storage container in a state in which sevoflurane vapor is present in the storage container in "step B," and then drain the liquid outside of the storage container. As a result, it has been revealed that washing can be achieved to such an extent that substantially none of sevoflurane and hydrophobic "polyether 1 and polyether 2" remains (at less than 1 ppm) in the washed container. Further, even after the repetitive reuse of the storage container, sevoflurane, polyether 1, and polyether 2 were not found to accumulate in the storage container (see the "Examples" below).

Further, the present inventors have attempted to conduct a washing step corresponding to "step B" while placing sevoflurane in a stringent environment in an intended manner in the storage container so as to forcibly convert a portion of sevoflurane into "polyether 1 and polyether 2" and storing the obtained "sevoflurane containing the polyethers dissolved therein at relatively high concentrations" in the storage container. As a result, the present inventors have found that it is possible to wash not only sevoflurane but also "polyether 1 and polyether 2" obtained as by-products away from the sevoflurane storage container using a "liquid containing water as a major component" to a level below a detection limit of 1 ppm for gas chromatography (FID). In particular, the present inventors have found that it is possible to use a "liquid substantially consisting of water" as the "liquid containing water as a major component" in a particularly preferable manner. That is, the present inventors have confirmed that sevoflurane can be drained outside of the system to an extent such that none of sevoflurane and "polyether 1 and polyether 2" can be detected (at a level below 1 ppm) by washing a storage container containing "sevoflurane containing polyether 1 and polyether 2 dissolved therein" with a "liquid substantially consisting of water" several times as described in the Examples below.

Sevoflurane is a fluorine-containing chain ether. Also, sevoflurane is a substance having low polarity and thus has low affinity for water. As disclosed in Patent Literature 5, sevoflurane can be mixed with a minute amount of water, thereby improving stability of sevoflurane, which is known as a specific effect. However, the upper limit of the amount of water that can be mixed with sevoflurane is about 1400 ppm. If the amount of water exceeds such level, water and sevoflurane tend to be strongly repulsive to each other.

Further, "polyether 1 and polyether 2" described above contain more carbon atoms than sevoflurane and therefore they have intensified hydrophobic characteristics. That is, each of polyether 1 and polyether 2 is a substance having greater polarity (stronger hydrophobicity) than sevoflurane.

In general, "water" is a liquid having very high polarity (permittivity of vacuum: about 80) and therefore it has low affinity for organic compounds (and especially compounds having low polarity and containing many carbon atoms). For such reason, it has been expected that "water" is obviously an inappropriate washing liquid for washing sevoflurane and its analogs. Specifically, it has been considered that when water is used as a washing liquid, sevoflurane and especially "polyether 1 and polyether 2" having lower polarity than sevoflurane would remain on the inner wall of a storage container.

Nevertheless, the present inventors have found that "sevoflurane analogs (polyether 1 and polyether 2)" remaining on the container inner wall can be effectively washed away with "water" that has been regarded as an inappropriate washing liquid (to such an extent that the analogs can be washed to a level meeting requirements for quality control of medicines). Even those skilled in the art have never expected such finding. One possible reason for the finding is that as sevoflurane can be dissolved in a small amount of water, "sevoflurane" remaining as vapor in the container somehow interacts with "water," allowing a mixture of sevoflurane and water to effectively wash away "polyethers" on the container inner wall. (After having achieved the present invention, the present inventors re-examined references and found that JP Patent No. 3612590 discloses that "polyether 1" forms an azeotropic mixture when mixed with butanol. Also, JP Patent No. 5244109 discloses an experimental example in which an organic compound composition including "polyether 1" was washed with water (Example 3); however, the content of "polyether 1" remained unchanged before and after water washing. Both references merely support that "polyether 1" is a substance having very low affinity for water.)

Note that a used "sevoflurane storage container" (i.e., a sevoflurane storage container subjected to the consumption step) is usually not exposed to a "harsh environment" during actual introduction of sevoflurane. It is therefore considered that sevoflurane in a "used container" is unlikely to be converted into its analogs such as "polyether 1 and polyether 2" described above. However, as sevoflurane is used as a medicine, quality control must always be carried out for the worst-case scenario. Specifically, it is essential to completely drain sevoflurane outside of the storage container by the time of filling sevoflurane for the next lot, even in case that sevoflurane is converted into polyether 1 and polyether 2 for some reason. That is, the washing means is indispensable to completely eliminate sevoflurane product-related risks and ensure quality of sevoflurane with certainty.

The present inventors have found that a "liquid containing water as a major component" and especially a "liquid substantially consisting of water" can be preferably used as washing means, thereby making it possible to stably carry out container washing many times while sufficiently ensuring quality assurance required for pharmaceutical products. Accordingly, economic burden to conventional washing of sevoflurane storage containers could be remarkably reduced.

According to the present invention, the expression "liquid containing water as a major component" refers to a "single-layer liquid containing 50% by mass or more of water." Specifically, such liquid is preferably either a "liquid forming a single layer of a mixture of water and a non-water liquid" or "liquid substantially consisting of water." A "liquid substantially consisting of water" is particularly preferable. That is, the present inventors have found that even a "liquid substantially consisting of water" can be used for container washing so that washing can be carried out to a level comparable to "rinsing with sevoflurane."

The washing operation used herein is not particularly limited. However, it is preferable to perform the operation by directly injecting a "liquid containing water as a major component" to the inner wall of a storage container using a spray nozzle. Particularly preferably, the temperature of the liquid is a relatively high temperature of 60°C to 90°C.

The present invention is also characterized by carrying out a step of introducing a drying gas into the storage container so as to drain the liquid remaining on the storage container inner wall together with the drying gas outside of the storage container (step C) after step B above.

Step C above allows a portion of the "liquid containing water as a major component" used for washing of the sevoflurane storage container, which is adhering to (remaining on) the container inner wall, to be drained together with the drying gas outside of the container, thereby drying the container inner wall. In addition, sevoflurane vapor might remain in a gas phase of the storage container after step B. However, such sevoflurane vapor can be completely drained outside of the container by carrying out step C. This allows the storage container to be suitable for refilling of sevoflurane for a new lot.

It is possible to produce a "pharmaceutical product of sevoflurane filled into a container " by conducting a step of filling sevoflurane for a new lot using a sevoflurane storage container subjected to the "step of washing a sevoflurane storage container (the 1st step)" involving steps A to C above.

Further, it is possible to stably store the produced "pharmaceutical product" in the subsequent "storage step "(the 3rd step).

The present invention is also advantageous for the following reasons. When "rinsing with sevoflurane" is performed without washing with a "liquid containing water as a major component," it is necessary to fill a container with sevoflurane for a new lot as soon as possible after "rinsing with sevoflurane" (usually within one month) from the viewpoint of step control. This is because a state in which "a relatively small amount of sevoflurane remains in a high-capacity storage container" differs more or less from a state in which "a storage container is filled with a predetermined volume of sevoflurane" upon product shipment in terms of environment as mentioned above, and it is preferable not to allow sevoflurane to remain in such state for an unnecessarily long period of time.

That is, if "rinsing with sevoflurane" is carried out, it is the best way to complete a step of filling the container with sevoflurane for a new lot as soon as the rinsing has been completed in terms of quality control.

Meanwhile, when washing with a "liquid containing water as a major component" is carried out according to the present invention, substantially no sevoflurane or by-product exists in the container. In this case, even if the container is filled with sevoflurane for a new lot after the container has been left for a long period of time after the completion of the washing step (e.g., 6 months), there would be no problems. Specifically, it is also possible to "wash the container" and "fill sevoflurane for a new lot" at different timings, thereby significantly improving the degree of freedom of each step.

As stated above, the present inventors have found that a sevoflurane storage container can be washed using a "liquid containing water as a major component," which is unexpectedly advantageous. This has led to the completion of the inventions of the present application.

Specifically, the present invention encompasses the following inventions.

### [Invention 1]

A method for washing a sevoflurane storage container, wherein
the storage container is a sevoflurane storage container, which is repeatedly used for a series of lots, the storage container having been subjected to a step of consuming at least a portion of sevoflurane filled into the storage container for a previous lot,
the method comprising the steps of:
creating a state in which at least sevoflurane vapor is present in the storage container (step A);
bringing a liquid containing water as a major component into contact with the inner wall of the sevoflurane storage container in the state in which sevoflurane vapor is present in the storage container after step A and draining the liquid outside of the storage container while the liquid remains liquid (step B); and
introducing a drying gas into the storage container so as to drain the liquid remaining on the inner wall of the storage container together with the drying gas outside of the storage container after step B (step C).

### [Invention 2]

The washing method according to Invention 1, wherein the state in which at least sevoflurane vapor is present in the storage container in step A is a state that is achieved by consuming substantially all liquid sevoflurane filled into the storage container in the step of consuming sevoflurane filled into the storage container for a previous lot and then hermetically sealing the storage container while allowing at least a portion of sevoflurane vapor to remain in a gas phase in the storage container.

### [Invention 3]

The washing method according to Invention 1, wherein the state in which at least sevoflurane vapor is present in the storage container in step A is a state that is achieved by consuming a portion of liquid sevoflurane filled into the storage container in the step of consuming sevoflurane filled into the storage container for a previous lot and then hermetically sealing the storage container.

### [Invention 4]

The washing method according to any one of Inventions 1 to 3, wherein the liquid containing water as a major component is a liquid substantially consisting of water.

### [Invention 5]

The washing method according to any one of Inventions 1 to 4, wherein in order to bring a liquid containing water as a major component into contact with the inner wall of the sevoflurane storage container in step B, the liquid is directly injected to the inner wall of the storage container using liquid injection means.

### [Invention 6]

The washing method according to Invention 5, wherein the temperature of the liquid containing water as a major component is 60°C to 90°C upon injection.

### [Invention 7]

The washing method according to any one of Inventions 1 to 6, wherein a part of or all of the inner wall of the storage container is made of at least one material selected from the group consisting of stainless steel, resin lining, and glass.

### [Invention 8]

The washing method according to Invention 7, wherein a part of or all of the inner wall of the storage container is made of stainless steel.

### [Invention 9]

The washing method according to any one of Inventions 1 to 8, wherein the drying gas in step C is dry air at 30°C to 150°C.

### [Invention 10]

The washing method according to any one of Inventions 1 to 9, wherein step C includes confirming that the drying gas discharged from the storage container has a dew point at or lower than a predetermined temperature.

### [Invention 11]

A method for washing a sevoflurane storage container, wherein
the storage container is a sevoflurane storage container, which is repeatedly used for a series of lots, the storage container having been subjected to a step of consuming at least a portion of sevoflurane filled into the storage container for a previous lot,
the method comprising the steps of:
creating a state in which at least sevoflurane vapor is present in the storage container by hermetically sealing the storage container while allowing at least a portion of sevoflurane vapor to remain in a gas phase in the storage container after consuming sevoflurane in the step of consuming sevoflurane filled into the storage container for a previous lot (step a);
directly spraying a liquid substantially consisting of water the inner wall of the storage container using a spray nozzle so as to bring the liquid into contact with the inner wall in a state in which sevoflurane vapor is present in the storage container after step a and then draining the liquid outside of the storage container while the liquid remains liquid (step b); and
introducing a drying gas into the storage container so as to drain the liquid remaining on the inner wall of the storage container together with the drying gas outside of the storage container after step b and confirming whether the dew point of the drying gas discharged from the storage container is at or lower than a predetermined temperature (step c),
wherein as a result of implementation of steps a to c, sevoflurane and its analog compounds (polyether 1 and polyether 2) expressed by the following formula are substantially not detected in the storage container:

(CF₃)₂CHO-CH₂-O-CH(CF₃)₂ <Polyether 1>

(CF₃)₂CHO-CH₂-O-CH₂-O-CH(CF₃)₂ <Polyether 2>

### [Invention 12]

The washing method according to Invention 11, wherein a part of or all of the inner wall of the storage container is made of stainless steel, and the temperature of the liquid substantially consisting of water is 60°C to 90°C.

### [Invention 13]

The washing method according to any one of Inventions 1 to 12, wherein step B or step b includes a step of washing the storage container using purified sevoflurane.

### [Invention 14]

A method for producing a pharmaceutical product, which is sevoflurane filled into a storage container, the method comprising filling a storage container washed by the method according to any one of Inventions 1 to 13 with sevoflurane for a new lot.

### [Invention 15]

The washing method according to Invention 14, wherein the liquid composition of sevoflurane filled into a storage container for a new lot is assayed by gas chromatography.

### [Invention 16]

A pharmaceutical product of sevoflurane filled into a storage container, which is produced by the method according to Invention 14 or 15.

### [Invention 17]

A method for storing sevoflurane, comprising the following 1st to 3rd steps:
the 1st step of washing a sevoflurane storage container by the method according to any one of Inventions 1 to 13;
the 2nd step of filling the storage container with sevoflurane for a new lot after washing; and
the 3rd step of storing the sevoflurane storage container after filling.

### ADVANTAGEOUS EFFECTS OF INVENTION

The present invention is advantageous in that it is possible to effectively wash a used "sevoflurane storage container" with an inexpensive liquid containing water as a major component. That is, washing can be carried out to such an extent that substantially none of sevoflurane and its analogs (polyether 1 and polyether 2) can be detected inside of a storage container.

Moreover, according to the present invention, it is possible to separately carry out the step of washing the storage container and the subsequent step of filling the storage container with sevoflurane, thereby allowing the degree of freedom of each step to increase.

### BRIEF DESCRIPTION OF DRAWINGS

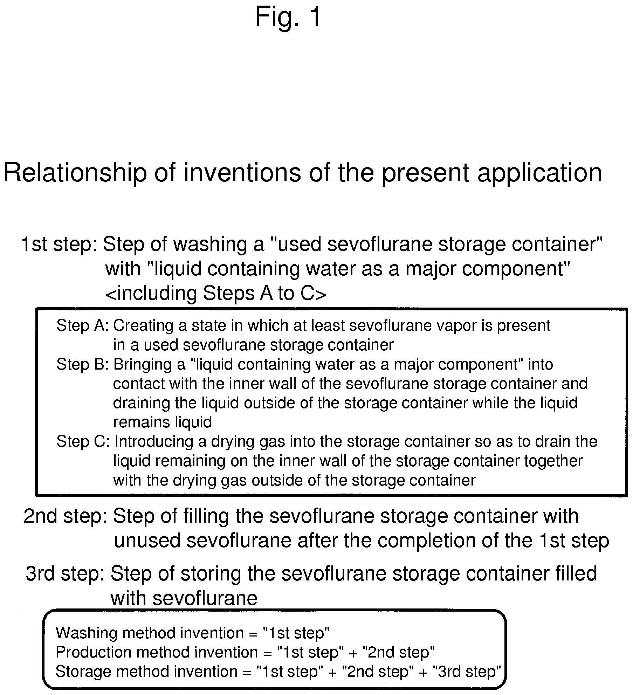

[Figure 1] Figure 1 illustrates a relationship of a "method for washing a used storage container containing sevoflurane," a "method for producing a pharmaceutical product of sevoflurane filled into a storage container," and a "method for storing sevoflurane."
[Figure 2] Figure 2 exemplifies a preferable embodiment of "step B" in the 1st step.
[Figure 3] Figure 3 shows a schematic view of "nozzle <a>" used in "step B" in the 1 st step.
[Figure 4] Figure 4 exemplifies a preferable embodiment of "step C" in the 1st step.

### DESCRIPTION OF EMBODIMENTS

The present invention is described in detail below. The scope of the present invention is not limited to the following descriptions. Therefore, various modifications can be made appropriately in embodiments other than the embodiments below without departing from the scope and spirit of the present invention.

This description includes part or all of the content as disclosed in the description and/or drawings of Japanese Patent Application No. 2016-215676, which is a priority document of the present application. In addition, all publications such as prior art documents, laid-open application publications, patent publications, and other patent literature cited herein are incorporated herein as reference.

Each of the inventions of the present application involves the following 1 st to 3rd steps (see Invention 17).
1st step: step of washing a "sevoflurane storage container" with a "liquid containing water as a major component"
2nd step: step of filling the storage container with sevoflurane for a new lot after the completion of the 1 st step
3rd step: step of storing the sevoflurane storage container filled with the sevoflurane after the 2nd step.

Among the inventions of the present application, the "washing method invention" is defined as including the "1st step" described above as a constituent feature. Specifically, the "1st step" includes "step A" to "step C."

The "production method invention" is defined as including the "1st step" and the "2nd step" as constituent features.

The "storage method invention" is defined as including the "1st step," the "2nd step," and the "3rd step" described above as constituent features.

The above steps are specifically described in the order starting from the "1st step." The constituent features and combinations thereof in the following embodiments are described as examples. Therefore, constituent features may be added, omitted, replaced, or modified without departing from the scope and spirit of the present invention. Moreover, the present invention is not limited to the embodiments below.

### Regarding the 1 st step

The 1st step is a step of washing a "sevoflurane storage container" with a "liquid containing water as a major component."

### [Regarding sevoflurane]

Any sevoflurane product widely used as a general anesthetic can be employed as sevoflurane in the present invention. A sevoflurane synthesis method is not particularly limited. However, sevoflurane produced by any of the methods disclosed in Patent Literature 1 to 5 can be preferably used.

### [Regarding "sevoflurane storage container"]

According to the present invention, a "sevoflurane storage container" that is an object to be washed (i.e., a used sevoflurane storage container) is a sevoflurane storage container which is repeatedly used for a series of lots, in which at least a portion of sevoflurane filled into the storage container for a previous lot has been consumed.

Sevoflurane is an ether and thus it is highly volatile. In view of this, a storage container is required to have a structure in which sevoflurane can be hermetically stored. The shape or material of the storage container is not particularly limited; however, the container body is preferably made of a metal such as stainless steel. In addition, a part of or all of the inner wall of the storage container is preferably made of at least one material selected from the group consisting of stainless steel (i.e., iron-chromium-nickel alloy), resin lining, and glass. In particular, a storage container having an inner wall made of stainless steel or resin lining is characterized by high physical strength and chemical durability (corrosion resistance), and therefore, it is suitable for storing sevoflurane under hermetically sealed conditions. A storage container having an inner wall made of stainless steel is particularly preferable because it has a simple structure and very high durability. Particularly preferable examples of stainless steel include austenite-based stainless steel products known as SUS304, SUS316, and SUS316L.

Sevoflurane is in the form of volatile liquid. For example, its vapor pressure is about 0.026 MPa at 25°C, about 0.041 MPa at 35°C, and about 0.061 MPa at 45°C. When liquid sevoflurane is introduced into a storage container filled with an inert gas (e.g., nitrogen), it is common to introduce the liquid into the container and seal the container, following which a portion of the liquid gradually evaporates into a gas phase in the storage container. Accordingly, the inside of the storage container is slightly pressurized if the temperature does not change in most cases. If the ambient temperature significantly decreases after filling, the inside of the storage container may become a negative pressure state (less than 1 atmosphere). It is therefore preferable for a sevoflurane storage container to have a structure durable for internal pressure variation (increase or decrease). Specifically, if a storage container is filled with sevoflurane under such conditions, the internal pressure would vary from about 0.08 MPa to 0.13 MPa. That is, a "container capable of bearing an internal pressure (absolute pressure) of 0.17 MPa" would be sufficiently able to cope with such pressure variation, which is preferable. There is no upper limit of pressure resistance for the container. However, if a "container capable of bearing an absolute pressure of 0.3 MPa or more" is used, design and maintenance would be complicated. In addition, as the internal pressure of a sevoflurane storage container usually does not exceed 0.3 MPa, it is not necessary to use a storage container capable of bearing an absolute pressure of 0.3 MPa or more in most cases.

According to the present invention, the "sevoflurane consumption step" is specifically a step of, for example, conducting quality evaluation of sevoflurane filled into a storage container, collecting sevoflurane from the storage container, and dispensing it as a medicine into glass ampules or the like. In this "consumption step," a dip tube for sevoflurane collection is inserted from the outside of a storage container into the liquid phase of sevoflurane in the storage container, and an inert gas (e.g., nitrogen) is introduced into the gas phase of the storage container via a different port for pressurization, thereby slightly pressuring the inside of the storage container. By doing so, it is possible to collect liquid sevoflurane through the dip tube.

In the above "consumption step," it is usual, but not necessary, to return a storage container to a sevoflurane manufacturer after the storage container becomes substantially empty as a result of "consumption" of a large portion of liquid sevoflurane filled into the storage container for a previous lot.

The inner volume of a sevoflurane storage container is not particularly limited. The sevoflurane storage container may have a size appropriate for transportation of a reasonably large amount of sevoflurane. For example, a storage container having a large inner volume of 100 dm³ to 10000 dm³ can be used. Such container may have a larger inner volume or a smaller inner volume. According to the present invention, a "sevoflurane storage container" having an inner volume of 300 dm³ to 1000 dm³ is particularly preferable.

### [Regarding "liquid containing water as a major component"]

According to the present invention, the "liquid containing water as a major component" used as a washing agent may be a "single-layer liquid containing 50% by mass or more of water." Specifically, it is preferably either a "liquid forming a single layer of a mixture of water and a non-water liquid" or a "liquid substantially consisting of water." A "liquid substantially consisting of water" is particularly preferable.

The "non-water liquid" used herein is a liquid capable of forming a single-layer liquid when mixed with water (e.g., alcohol or acetone). In addition, a liquid that has a low affinity for water but forms a single-layer liquid when mixed in a very small amount with water can be used as the "non-water liquid" in the present invention as long as the liquid composition allows such liquid to form a single-layer liquid when mixed with water.

Examples of the "non-water liquid" include, but are not particularly limited to, known hydrophilic solvents such as methanol, ethanol, 2-propanol, acetone, acetonitrile, tetrahydrofuran, ethylene glycol, trimethylamine, and triethylamine. If a liquid mixture comprising "water and a non-water liquid" is used, the mass of water may be 50% by mass or more, preferably 80% by mass or more, and 90% by mass or more of the total mass of the liquid mixture. Note that when a substance other than sevoflurane is mixed as a "non-water liquid" with water so that the mixture is used as a washing solvent, it is necessary to carry out step C described below until the "non-water liquid" becomes not detected. This may cause burden because the content of the "non-water liquid" must be set as a test item.

In consideration of the above, the present inventors have found that the a "liquid substantially consisting of water" can be preferably used as a "liquid containing water as a major component." It is therefore particularly preferable to use a "liquid substantially consisting of water" as a "liquid containing water as a major component" in the 1 st step of the present invention.

Type of water used herein is not particularly limited from the technical viewpoint. It is possible to appropriately use normal tap water, ion-exchange water, distilled water, reverse osmosis water (RO water), or the like. Note that as sevoflurane is a substance that may be used as a medicine, it is preferable to use containing fewer impurities. It is particularly preferable to use ion-exchange water or distilled water. In a particularly preferable embodiment, ion-exchange water having an electric conductivity at or lower than a predetermine level of 1 µS/cm, which is obtained by allowing water to pass through ion-exchange resin, is used.

### [Regarding step A]

Step A in the 1 st step is a step of creating a state in which at least sevoflurane vapor is present in a sevoflurane storage container. The storage container that is an object to be washed is described in detail above. Once step A is carried out, sevoflurane molecules are continuously brought into contact with the inner wall of the storage container, thereby making it possible to obtain effects of washing with a "liquid containing water as a major component" in the subsequent step (step B) with certainty. In case that step A is not carried out and the container inner wall is dried, it becomes difficult to expect sufficient washing effects even if washing is carried out using a "liquid containing water as a major component" in the subsequent step (step B). That is, step A plays an important role in the present invention.

Means of creating the "state in which at least sevoflurane vapor is present in a sevoflurane storage container" in step A is not particularly limited. This means is explained in the "1st embodiment" to the "3rd embodiment" below.

### (1) 1st embodiment

In step A, the "1st embodiment" corresponds to a technique of collecting almost all liquid sevoflurane in a "step of consuming" sevoflurane filled into a storage container for a previous lot and then hermetically sealing the storage container while allowing sevoflurane vapor to remain in a gas phase in the storage container. Specifically, when almost all liquid sevoflurane is consumed (collected) in the "step of consuming" sevoflurane, a large amount of sevoflurane vapor still remains in a gas phase in the container. For instance, as sevoflurane vapor pressure is about 0.026 MPa at 25°C under atmospheric pressure, when almost all sevoflurane present in a liquid phase in a 500-dm³ container is collected, about 1 kg of sevoflurane would remain in a gas phase in the container, provided that loss of sevoflurane in the gas phase is negligible.

The present inventors have found that the amount of "sevoflurane vapor in a gas phase" in step A does not necessarily correspond to saturated vapor pressure. Specifically, sevoflurane may be present at 0.0026 MPa (10% of saturated vapor pressure at 25°C) or more, preferably 0.010 MPa (40% of saturated vapor pressure at 25°C) or more, and more preferably at 0.020 MPa (80% of saturated vapor pressure at 25°C) or more in the gas phase at 25°C.

Usually, it is possible to efficiently collect sevoflurane from a storage container filled with sevoflurane by inserting a dip tube into a liquid phase of sevoflurane, slightly pressuring the inside of the container via a different port for pressurization, and obtaining liquid sevoflurane via the dip tube while pressurization. When sevoflurane is obtained in such manner, even if substantially all liquid sevoflurane is collected, substantially saturated sevoflurane vapor remains in the container. This is because specific gravity of sevoflurane vapor is much greater than that of air or nitrogen and therefore sevoflurane vapor remains in the lower part (bottom) of the storage container in principle. Therefore, it is possible to keep "sevoflurane in an amount substantially corresponding to saturated sevoflurane vapor" intact in the storage container by closing the dip tube immediately (within, for example, 5 minutes and preferably 1 minute) after the sevoflurane consumption step without opening the lower part of the container or introducing an inert gas into the container (or it is also possible to hermetically close the storage container immediately after removing the dip tube). By carrying out this operation, it is possible to maintain the aforementioned "state in which the amount of sevoflurane vapor corresponds to 0.0026 MPa (10% of sevoflurane vapor pressure) at 25°C is present" with certainty.

### (2) 2nd embodiment

Another technique for step A (the 2nd embodiment) is a technique of consuming (collecting) a portion of sevoflurane in the step of consuming sevoflurane filled into a storage container for a previous lot (in other words, keeping a portion of sevoflurane in the liquid form in the storage container) and hermetically sealing the storage container. This technique allows "liquid sevoflurane" to remain in the storage container, resulting in the establishment of vapor-liquid equilibrium. Accordingly, the storage container can be filled with saturated sevoflurane vapor.

Specifically, in the above embodiment, it is possible to employ a technique of adjusting the position of a dip tube to be inserted into liquid sevoflurane in the sevoflurane consumption step so as not to consume (collect) a portion of the liquid phase of sevoflurane, a technique of injecting a predetermined volume of sevoflurane into the container in an intended manner when confirming the consumption of liquid sevoflurane in the sevoflurane consumption step," or the like.

In the "2nd embodiment," as long as the sevoflurane storage container is left at a temperature equivalent to (or below) the temperature in the "sevoflurane consumption step (collection step)," the liquid phase of sevoflurane is stably present in the container (that is to say, vapor-liquid equilibrium is established). It is therefore possible to maintain a state in which the container inner wall is in contact with "saturated vapor" of sevoflurane.

### (3) 3rd embodiment

As another technique for step A (the 3rd embodiment), a technique of purging all sevoflurane vapor remaining in the storage container for some reason after the consumption step for a previous lot and then additionally supply sevoflurane from the outside into the container" can also be employed. However, it is not advantageous in practice to purge sevoflurane and then additionally supply sevoflurane from the outside. As stated above, a large amount of sevoflurane is required to allow a predetermined volume of sevoflurane vapor to be present in a gas phase in a high-capacity storage container. This may result in wasteful use of sevoflurane. For such reason, the technique for the "3rd embodiment" is not the best technique in the present invention.

Those skilled in the art can appropriately determine whether to carry out the "1st embodiment" or the "2nd embodiment." In the case of the "2nd embodiment," although it is necessary to allow excess sevoflurane to be present in the storage container, it is possible to allow saturated sevoflurane vapor to be stably present in the container. Meanwhile, in the case of the "1st embodiment," as substantially all liquid sevoflurane is collected, sevoflurane vapor pressure in the container is usually below (or slightly lower than) saturated vapor pressure.

However, as stated above, the present inventors have found that it is certainly possible to allow the inner wall of the storage container to have affinity for sevoflurane even in the case of the "1st embodiment." In view of the maximum prevention of wasteful use of sevoflurane, the "1st embodiment" is considered more preferable.

When step A is conducted by any of the techniques for the 1st to 3rd embodiments, it is preferable to create a state in which sevoflurane vapor is present in a gas phase in the container in step A and maintain the state for at least 60 minutes in order to allow the container inner wall to have affinity for sevoflurane vapor. It is further preferable to maintain the state for 24 hours (one day). As is apparent from the above explanation, when the "sevoflurane consumption step" is completed and the container is hermetically sealed, "step A" is usually completed in the present invention. The used sevoflurane storage container in such state is returned (transported) to a manufacturer. Therefore, in general, after the sevoflurane consumption step, a requirement of "creating a state in which sevoflurane vapor is present in a gas phase in a sevoflurane storage container and then maintain the state for 1 hour (preferably 24 hours)" is satisfied without carrying out a special operation as long as the container is hermetically sealed after the sevoflurane consumption step.

### [Regarding step B]

In step B, after the completion of step A, a "liquid containing water as a major component" is brought into contact with the inner wall of the sevoflurane storage container while sevoflurane vapor is present in the storage container and then the liquid is drained outside of the storage container drain. As a result of step B, sevoflurane in the storage container is washed away from the storage container with the use of the liquid. In case that sevoflurane contains "polyether 1 and polyether 2," these analogs can be drained together with sevoflurane from the storage container with certainty. This is surprisingly advantageous.

In the present invention, it is preferable to carry out step A so as to create a state in which sevoflurane vapor is present in the storage container in advance and then carry out step B while maintaining such state (that is to say, while the inner wall is in into contact with sevoflurane vapor). By doing so, it is possible to achieve significant washing performance in step B of the present invention. Usually, a special operation for carrying out step B is not necessary. It is only necessary to provide a small port (inlet) for introducing a "liquid containing water as a major component" after step A to the storage container, provide different depressurization means, and immediately start to introduce the liquid. Accordingly, it becomes possible to replace the "state in which sevoflurane vapor is in contact with the inner wall" achieved in step A by the "state in which a liquid containing water as a major component is in contact with the inner wall" in step B in a continuous manner.

When a "liquid containing water as a major component" is brought into contact with the inner wall of the sevoflurane storage container while sevoflurane vapor is present in the storage container in step B of the present invention, it means that sevoflurane vapor remains in the storage container at the beginning of bringing the liquid into contact with the inner wall of the storage container. It is also preferable for sevoflurane vapor to be present in the container thereafter while washing with the liquid is continued. In particular, in the case of the "2nd embodiment" described below, the amount of liquid used can be reduced, resulting in a decrease in the rate of draining sevoflurane outside of the system. Accordingly, sevoflurane vapor tends to remain in the container while the liquid is brought into contact with the inner wall, which is preferable.

If liquid sevoflurane remains in the storage container when the aforementioned step A is completed (i.e., in the case of step A in the 2nd embodiment described above), liquid sevoflurane may be drained from the liquid drain port (drainpipe) at the lower part of the storage container before the start of step B. In particular, when the amount of remaining liquid sevoflurane is large, step B is started after liquid sevoflurane is drained, which is preferable because wasteful use of sevoflurane can be prevented.

It is usually difficult to "introduce a liquid containing water as a major component" in step B while keeping the storage container completely hermetically sealed (because of an increase in internal pressure). It is therefore preferable to apply depressurization means to the storage container.

As stated above, according to the present invention, the "liquid containing water as a major component" needs to be a "single-layer liquid containing 50% by mass or more of water" and preferably a "liquid substantially consisting of water." As stated above, the main feature of the present invention is that a sevoflurane storage container can be washed effectively even with the use of a "liquid substantially consisting of water." This feature makes it possible to avoid complexity of control/management of a residual "non-water solvent" in the container.

The expression "liquid substantially consisting of water" refers to a liquid composed of a substance comprising, for example, water molecules that account for 99.9% by mass of the substance. As stated above, it is particularly preferable to use ion-exchange water or distilled water. With the use of such water, the requirement that "liquid is composed of a substance comprising water molecules that account for 99.9% by mass of the substance" is sufficiently satisfied.

The temperature of the "liquid containing water as a major component" used in step B is not particularly limited. It is particularly preferable to use a liquid within a temperature range of 60°C to 90°C for warm or hot water. If the temperature is excessively low, sufficient washing effects cannot be exerted, which may result in an increase in the amount of the liquid necessary for washing. Meanwhile, if the temperature exceeds 90°C, it becomes complicated to handle the liquid at an excessively high temperature.

In step B, it is desirable to bring the "liquid containing water as a major component" into contact with the entire inner wall of the sevoflurane storage container in order to remove chemical species left on the container inner wall with sufficient washing. A technique for such purpose is not limited; however, representative techniques are described in the 1st embodiment and the 2nd embodiment below

### (1) 1st embodiment

Step B of the "1st embodiment" corresponds to a technique of completely filling a storage container with a "liquid containing water as a major component," if preferred, stirring the liquid for a predetermined time period, and draining the liquid from the drainpipe. This technique does not require a complicated operation and allows a "liquid containing water as a major component" to come into contact with the entire inner wall of the storage container with certainty. That is, in this embodiment, sufficient washing can be carried out by a convenient operation. The technique of completely filling a storage container with a "liquid containing water as a major component," stirring the liquid for a predetermined time period, and draining the liquid is repeated preferably at least 3 times and more preferably at least 5 times.

This technique requires completely filling the storage container with the "liquid containing water as a major component." In this case, an exhaust port is usually provided to the top part of the storage container.

The "1st embodiment" is advantageous in that washing can be carried out by a convenient operation. Meanwhile, it is disadvantageous in that the amount of the "liquid containing water as a major component" must be adjusted to the inner volume of the storage container in order to carry out step B once. In particular, in order to wash, for example, a high-capacity storage container with a volume of 500 dm³, a large amount of the "liquid containing water as a major component" is necessary, which might be economically disadvantageous.

### (2) 2nd embodiment

Step B of the "2nd embodiment" corresponds to a technique of directly injecting a "liquid containing water as a major component" to the entire inner wall of a storage container by liquid injection means. Examples of liquid injection means include conventional means such as a spray nozzle or a shower. Although this technique requires a certain operation, it is an excellent technique because sufficient washing can be carried out, although the amount of water used is significantly smaller than that used in the 1st embodiment. In addition, as stated above, as it is possible to reduce the amount of water used in this embodiment, sevoflurane vapor tends to remain in a gas phase for long time. Even if "polyether 1 and polyether 2" are present, these substances can be removed by powerful washing, which is advantageous.

In the "2nd embodiment," it is particularly preferable to carry out an operation of injecting the liquid from the spray nozzle to allow droplets of the liquid injected from the nozzle to directly collide against the container inner wall while gradually changing the angle of the nozzle until the droplets have collided against the entire container inner wall.

When "water" is used as a solvent, it tends to form a mass with strong intermolecular hydrogen bonds. When the nozzle injects the "liquid containing water as a major component" to a specific site alone (e.g., the top part inside of the storage container), liquid droplets falling inside of the container form a line at the specific site and fail to come into contact with the entire inner wall of the container. This failure could be prevented by allowing the nozzle to inject the liquid so that liquid droplets directly collide against the entire container inner wall. Accordingly, it is possible to bring the "liquid containing water as a major component" into contact with the entire inner wall of the storage container.

A washing liquid injection method is not particularly limited. A wide range of manual or automatic methods can be employed. However, as stated above, it is common to use a container made of a non-transparent material such as stainless steel as a sevoflurane storage container. In such case, it is usually difficult to "inject water toward the entire inner surface of the container while visually observing the inside of the container."

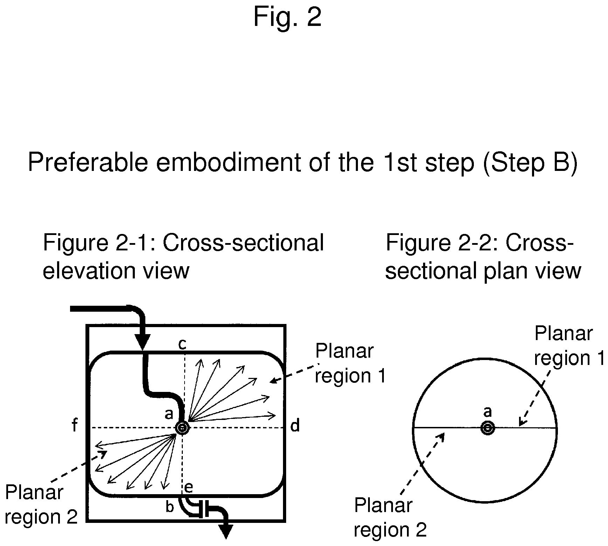

In consideration of the above, it is particularly preferable to conduct step B in the "2nd embodiment" using a "horizontally rotatable spray nozzle" as shown in Figure 2. Figure 2-1 is a cross-sectional elevation view of the sevoflurane storage container viewed from the horizontal direction. Figure 2-2 is a cross-sectional plan view of the same viewed from the top face of the storage container.

A "horizontally rotatable spray nozzle <a>" is placed near the center of the inside of the storage container (e.g., at the center position viewed from either the horizontal direction or the vertical direction). The nozzle <a> is connected to an external tank, from which the "liquid containing water as a major component" is supplied to the nozzle <a>. The nozzle <a> functions to inject water supplied in the direction "obliquely upward to the right by an angle of 90° with respect to the nozzle <a>" and in the direction "obliquely downward to the left by an angle of 90° with respect to the nozzle <a>" at the same time. As a result of water injection in the direction "obliquely upward to the right by an angle of 90° with respect to the nozzle <a>," the water flow forms a "planar region 1." As a result of water injection in the direction "obliquely downward to the left by an angle of 90° with respect to the nozzle <a>," the water flow forms a "planar region 2." The "planar region 1" and the "planar region 2" are joined via the nozzle <a> and they are on a plane (single plane) perpendicular to a straight line that connects an observer and the nozzle <a> in Figure 2.

In Figure 2, <b> denotes a drain port (or drainpipe) for draining liquid, which also functions to depressurize in the "2nd embodiment." Therefore, the drain port <b> should be opened during liquid injection.

In Figure 2-1, <c> and <d> respectively denote the upper end and the lower end of a curve that is formed when the "planar region 1" intersects with the container inner wall. The lower end <c> overlaps the top center point of the storage container. Meanwhile, <e> and <f> respectively denote the upper end and the lower end of a curve that is formed when the "planar region 2" intersects with the container inner wall. The upper end <e> overlaps the bottom center point of the storage container.

Figure 2-1 shows that the washing liquid injected from the nozzle <a> passes through the "planar region 1" and the "planar region 2" and directly collides against the curve extending from <c> to <d> and the curve extending from <e> to <f>.

Next, the nozzle <a> is rotated by 360° in the horizontal direction at a constant rotation rate, during which the state in which water is injected in the direction "obliquely upward to the right by an angle of 90° with respect to the nozzle <a>" and in the direction "obliquely downward to the left by an angle of 90° with respect to the nozzle <a>" at the same time shown in Figure 2-1 is maintained. As a result, a curve that corresponds to the curve extending from <c> to <d> and a curve that corresponds to the curve extending from <e> to <f> are continuously formed in the direction along the inner wall in the storage container in a 360-degree view. Specifically, as a result, the sprayed "liquid containing water as a major component" directly collides against the entire container inner wall. Accordingly, the inside of the container can be sufficiently washed using the "liquid containing water as a major component" in an amount that is significantly smaller than that used in the "1st embodiment."

It is preferable that the pressure of water injected from the nozzle <a> always exceed ordinary pressure because it is necessary to allow the "liquid" to directly collide against the entire container inner wall with certainty. For a high-capacity storage container with a volume of 500 dm³, the pressure is preferably 0.2 to 1 MPa (absolute pressure). The horizontal rotation rate of the nozzle <a> is not particularly limited; however, the rotation is performed within a cycle of 0.1 second to 10 seconds in one preferable embodiment. Note that the rotation rate of nozzle <a> is not necessarily critical. As long as the "liquid" is sprayed over the entire container inner wall, the rotation rate is not particularly limited (the rotation of nozzle <a> generates a driving force of "liquid injection pressure").

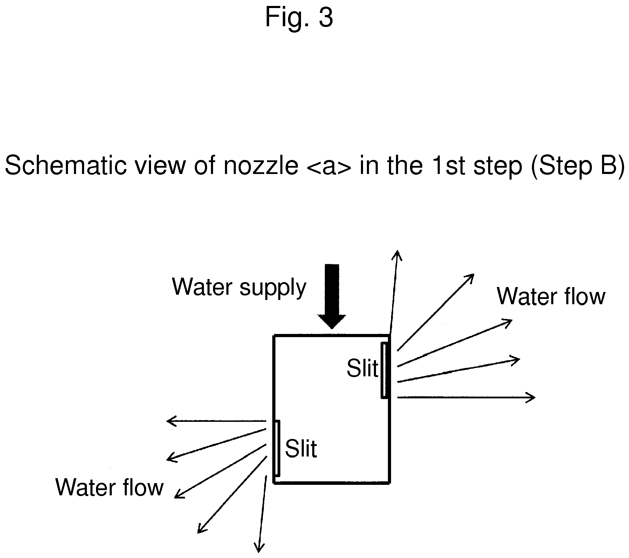

The nozzle <a> having the above liquid injection function is not particularly limited. However, it is preferable to use a commercially available nozzle such as "CERJET (registered trademark) (H. Ikeuchi & Co., Ltd.)." Figure 3 schematically shows such nozzle <a>. As shown in Figure 3, a slit is formed on faces of the nozzle, which are opposed each other. Supplied water is injected through each slit. Water is injected to form a "planar region 1" from one slit, while water is injected to form a "planar region 2" from the other slit (the nozzle <a> horizontally rotates to generate a driving force of "liquid injection pressure" in the example illustrated in Figure 3). Water is supplied from an external reservoir. The position, length, and width of each slit are determined so that the liquid is injected in desired directions. When the nozzle <a> having the structure shown in Figure 3 is used, the slit width is preferably 0.4 mm to 1 mm (and particularly preferably 0.5 to 0.7 mm).

In addition, immediately after the spray nozzle is prepared, a transparent material such as acrylic is used around a flange so that the inside of the container can be monitored from the outside, or a monitor camera is provided inside of the container. This is because it is preferable to confirm whether the liquid is allowed to collide against the entire container inner wall by the technique in the "2nd embodiment." Accordingly, it is possible to finely adjust how to inject the liquid or how to rotate the nozzle in the most appropriate manner. Once the optimum operation conditions are determined, the operation conditions are validated and reproduced in the subsequent operations, thereby making it possible to conduct effective washing without frequently monitoring the inside of the container.

When the "2nd embodiment" is applied, a method for continuously "injecting the liquid by liquid injection means" may be carried out. Meanwhile, it is also possible to employ a method comprising repeating an operation of injecting the liquid for a predetermined time period (e.g., 15 seconds), waiting a predetermined time period (e.g., 30 seconds), and restarting injection at a timing after most of liquid present on the inner wall has been drained from the drainpipe. In order to save a washing liquid, the latter is preferable. When the latter method is carried out for "injecting the liquid by liquid injection means," the liquid being divided into, for example, 10 batches, it is possible to sufficiently wash a 500-dm³ container using preferably 30 dm³ to 500 dm³ and particularly preferably 50 dm³ to 200 dm³ of water in total.

Also when step B is carried out using the "liquid substantially consisting of water" in the "2nd embodiment," the necessary amount of the liquid is usually 30 dm³ to 500 dm³ and preferably 50 dm³ to 200 dm³ for a 500-dm³ container. As stated above, it is possible to obtain excellent washing effects with a fewer amount of washing liquid by injecting the liquid divided into small amounts for a plurality of times rather than injecting the total amount of the liquid at once. Therefore, the "amount of water" itself is not a critical factor that constitutes the present invention. Specifically, how to determine the amount of "liquid substantially consisting of water" and the number of times of washing depends on the material or shape of the storage container, meaning that there are different optimum conditions. Those skilled in the art can optimize the above conditions based on their technical knowledge. It is required to validate and comply with the optimized conditions for carrying out the present invention.

One option for the validation described above is to carefully pour a predetermined volume of methanol (note that sevoflurane and its analog (polyether) can be easily dissolved in methanol) into a storage container after carrying out step B in the "2nd embodiment" in a predetermined manner so as to bring methanol into contact with the entire inner wall of the storage container, collect the methanol wash liquid via a drainpipe provided to the lower part of the storage container after the elapse of a predetermined time period, and analyze the liquid by gas chromatography. More specifically, given that the inner volume of the storage container is 100, the volume of methanol to be poured is preferably 0.1 to 0.5 (and particularly preferably 0.2 to 0.4). After the introduction of methanol, the methanol wash liquid (usually in an amount that accounts for about 50% of the amount of methanol introduced) is collected as much as possible via the drainpipe after the elapse of 1 hour, for example. The collected methanol wash liquid can be assayed by gas chromatography. The present inventors confirmed that after validation of the optimum conditions, as long as step B was carried out in strict compliance with the conditions, sevoflurane and its analogs (polyether 1 and polyether 2) were not detected at a level of the detection limit (1 ppm) in the subsequent steps as a result of collection and analysis of the methanol wash liquid.

In most cases, it is possible to "drain the washing liquid" via a liquid drain port (drainpipe) provided to the lower part of the storage container regardless of whether step B is carried out in the "1st embodiment" or the "2nd embodiment." In the case of the "1st embodiment," it is necessary to completely close the drainpipe while injecting the "liquid containing water as a major component." In this case, depressurization is carried out via an opening formed on the upper part of the storage container. Meanwhile, in the case of the "2nd embodiment," the drainpipe (corresponding to <b> in Figure 2-1 in the 2nd embodiment) may be opened during washing liquid injection. In this case, the wash liquid that has been brought into contact with the inner wall of the storage container is directly drained from the drainpipe, which also serves as depressurization means. In addition, in order to avoid emission of sevoflurane vapor as much as possible so as to allow sevoflurane vapor to be present in the storage container for long time, one option is to provide a depressurization port to the upper part of the storage container and open the depressurization port while closing the drainpipe during washing liquid injection. Those skilled in the art can select such option according to need.

Step B is a step of washing a sevoflurane storage container using a "liquid containing water as a major component" as a washing solvent. However, it is also possible to carry out "washing with a liquid containing water as a major component" in combination with "washing with purified sevoflurane serving as a washing liquid (rinsing with sevoflurane)" according to need within the scope of the present invention. As stated above, there is no problem to use a "liquid containing water as a major component" (and particularly preferably a "liquid substantially consisting of water") for washing of the storage container itself in step B. One of the important findings of the present invention is that "polyether 1 and polyether 2" would not be concentrated in the storage container even after washing is carried out a plurality of times (for plurality of lots). Therefore, it is usually not necessary to combine "rinsing with sevoflurane" and washing with sevoflurane.

In a case in which "rinsing with sevoflurane" is carried out in step B, the order of "rinsing with sevoflurane" in step B is not particularly limited. However, when "rinsing with sevoflurane" is carried out after washing with a "liquid containing water as a major component," a small amount of liquid sevoflurane remains in a storage container after washing. In view of this, it is preferable to carry out the filling step (the 2nd step) as soon as possible after "rinsing with sevoflurane" (e.g., within one month at room temperature).

Step B and the subsequent step C (of introducing a drying gas) may be conducted alternately a plurality of times. If the two steps are conducted alternately a plurality of times, it would result in complexity of the steps. The present inventors have found that it is not necessary to conduct both steps a plurality of times in most cases because sufficient effects can be obtained by conducting steps B and C alternately once.

### [Regarding step C]

Step C is a step of introducing a drying gas into the storage container after step B and draining the liquid remaining on the inner wall of the storage container together with the drying gas outside of the storage container. By conducting step C, it is possible to smoothly drain a portion of the "liquid containing water as a major component" used in step B, which is adhering to (remaining on) the container inner wall, together with the drying gas outside of the container. This operation allows a portion of the "liquid remaining on the inner wall of the storage container" to evaporate so as to be drained outside of the container. A portion of the liquid remains liquid and is directly drained outside of the container. In addition, even if a small amount of sevoflurane vapor remains in a gas phase in the container after step B (note that sevoflurane vapor has been substantially completely drained outside of the container after step B in many cases), sevoflurane vapor is drained together with the flowing drying gas outside of the container in step C.

That is, it is possible to renew a sevoflurane storage container suitable for filling sevoflurane for the next lot in step C.

As stated above, it is possible to repeatedly conduct steps B and C alternately a plurality of times. Meanwhile, steps B and C may be conducted once in most cases. In addition, it is obviously understood that if step B is conducted after step C, step C must be conducted again after step B, thereby draining liquid components remaining on the container inner wall outside of the container.

As stated above, when sevoflurane is shipped as a product, a small amount of water is added as a stabilizer to sevoflurane in many cases. It should be noted that the amount of "water added as a stabilizer" must be strictly controlled for sevoflurane that is synthesized and purified for the next lot. It is therefore very important to completely drain "water used for washing of a sevoflurane storage container" outside of the storage container in step C in consideration of quality assurance of sevoflurane as a medicine.

The type of a drying gas used in step C is not particularly limited. However, dry air is preferably used. It is also possible to use dry nitrogen or dry argon, which tends to be chemically inactive. However, it is enough to use inexpensive dry air in the present invention because sevoflurane and sevoflurane analogs, which are present in the liquid form on the inner wall, are completely drained outside of the storage container in step B, which is particularly preferable.

The way of obtaining dry air is not particularly limited. However, for example, it is convenient and preferable to pressurize air using a compressor so as to turn moisture into liquid, collect a residual gas phase, and allow the gas phase to pass through a "dehumidifier containing, as an active ingredient, a drying agent such as zeolite or oxidized aluminium" for further dehumidification. The dryness of a drying gas is not particularly limited. However, if the dryness of a drying gas is higher, step C can be completed within a shorter period of time. In general, the dew point of a drying gas is preferably -30°C or less and more preferably -40°C or less.

It is possible to introduce a drying gas at ordinary temperature. However, it is preferable to introduce a heated drying gas to save time. For example, a drying gas is introduced at preferably 30°C to 150°C and more preferably 40°C to 100°C.

The way of introducing a drying gas in step C is not particularly limited. However, as the specific gravity of sevoflurane vapor is greater than 1, it is preferable to introduce a drying gas from the upper part of a storage container and drain the drying gas from the lower part thereof in step C. For instance, in a case in which a washing liquid has been introduced from an inlet at the upper part of a storage container in step B, the inlet may be used as an inlet for a drying gas in step C. It is also possible to use a liquid drain port (drainpipe) used in step B as a drying gas discharge port in step C.

The flow rate of a drying gas in step C is not particularly limited. However, the flow rate is preferably 100 dm³/minute to 200 dm³/minute and more preferably 150 dm³/minute to 200 dm³/minute in a 500-dm³ container.

The introduction time may vary depending on conditions. However, the introduction time is typically 60 to 300 minutes when a "liquid substantially consisting of water" is used in step B and hot air at 30°C to 150°C is introduced in step C.

As in the case of step B, the optimum conditions for step C would vary depending on the material or shape of container. It is desirable for those skilled in the art to optimize and validate the conditions based on their technical knowledge. Once the optimum conditions are established, it is possible to smoothly conduct step C in compliance with the established conditions.

Step C may comprise determining the dew point of a drying gas discharged from the storage container after the above steps and confirming whether the dew point is at or below a predetermined temperature (e.g., -20°C when using a drying gas having a dew point of -30°C or less or -30°C when using a drying gas having a dew point of -40°C or less). In this case, it is possible to directly determine the dew point of a drying gas discharged from the storage container while introducing the drying gas. It is further preferable to temporarily suspend the continuous introduction of a drying gas, hermetically seal the container, inject a dry compressed gas into the container, keep the container pressurized for a predetermined time period, and purge the drying gas, thereby determining the dew point of the purged drying gas. It is possible to confirm with certainty whether there is no moisture deep inside of the inner wall in the latter case. Even after validation of the conditions for step C, it is preferable to determine the dew point upon the completion of step C for all batches. This is because step C is a step immediately before the "2nd step" of filling a container with sevoflurane for a new lot.

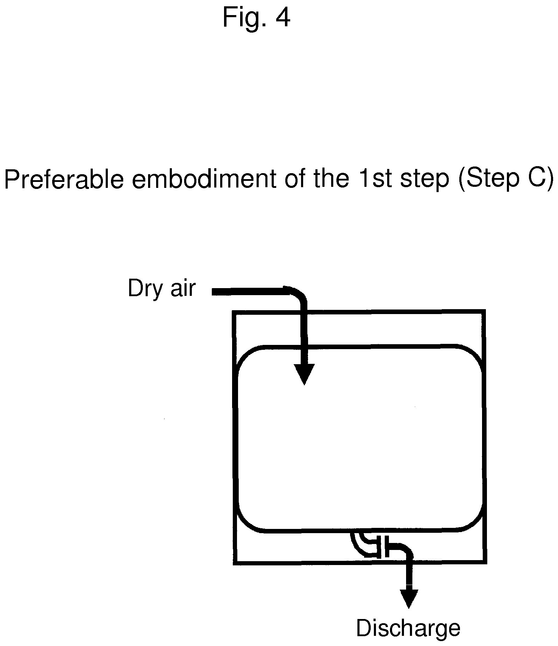

Figure 4 shows a preferable embodiment of step C.

### [Particularly preferable embodiment of the 1st step]

It is possible to conduct the 1st step of the present invention in a particularly preferable manner by a method including a combination of steps a to c described below. Specifically, such method is a method for washing a sevoflurane storage container, wherein the storage container is a sevoflurane storage container, which is repeatedly used for a series of lots, the storage container having been subjected to a step of consuming at least a portion of sevoflurane filled into the storage container for a previous lot, the method comprising the steps of:
creating a state in which at least sevoflurane vapor is present in the storage container by hermetically sealing the storage container while allowing at least a portion of sevoflurane vapor to remain in a gas phase in the storage container after consuming sevoflurane in the step of consuming sevoflurane filled into the storage container for a previous lot (step a);
directly spraying a liquid substantially consisting of water the inner wall of the storage container using a spray nozzle so as to bring the liquid into contact with the inner wall in a state in which sevoflurane vapor is present in the storage container after step a and then draining the liquid outside of the storage container while the liquid remains liquid (step b); and
introducing a drying gas into the storage container so as to drain the liquid remaining on the inner wall of the storage container together with the drying gas outside of the storage container after step b and confirming whether the dew point of the drying gas discharged from the storage container is at or lower than a predetermined temperature (step c).

As a result of steps a to c, sevoflurane and its analogs (polyether 1 and polyether 2) become substantially not detected in the storage container.

In addition, in this embodiment, it is particularly preferable that a part of or all of the inner wall of the storage container be made of stainless steel and the temperature of the "liquid substantially consisting of water" be 60°C to 90°C.

According to the present invention, the "state in which sevoflurane and its analogs (polyether 1 and polyether 2) are substantially not detected in the storage container" means as follows. When a sevoflurane storage container washed by the washing method of the present invention is filled with sevoflurane for a new lot (the 2nd step) so as to prepare a pharmaceutical product, polyether 1 and polyether 2 derived from the storage container are not substantially contained in the product. In addition, polyether 1 and polyether 2 do not substantially increase after the repetitive use of the sevoflurane storage container for a plurality of lots. Accordingly, sevoflurane product specifications can be stably satisfied.

More specifically, the above state means that when the contents of polyether 1 and polyether 2 in sevoflurane immediately before filling a storage container with the sevoflurane for a new lot are determined to be α1 (ppm) and α2 (ppm) and the contents of polyether 1 and polyether 2 in sevoflurane immediately after filling the storage container with the sevoflurane are determined to be β1 (ppm) and β2 (ppm), both (β1-α1) and (β2-α2) are less than 1 ppm. Usually, both α1 (ppm) and α2 (ppm) often become "less than 1 ppm" during production of sevoflurane. In this case, both β1 (ppm) and β2 (ppm) become "less than 1 ppm."

However, another option for conveniently confirming whether polyether 1 and polyether 2 are removed is to wash the inside of a storage container with a predetermined volume (e.g., container inner volume : organic solvent volume = 100 : 0.1 to 0.5) of an organic solvent capable of dissolving sevoflurane (e.g., methanol or diethyl ether) and determine whether or not polyether 1 and polyether 2 are detected in the wash liquid at a detection limit of 1 ppm for gas chromatography (FID). The past experience shows that if polyether 1 and polyether 2 are not detected in the above manner, it may be considered that the washing operation has been smoothly done (see the Examples below).

Another option is to extract a wash liquid collected in each washing operation in step B or b with a predetermined volume of diethyl ether so as to quantitatively determine polyether 1 and polyether 2 as explained in the Examples below. In this case, it is possible to confirm "washing effects of each washing operation" (see the Examples below).

### Regarding the 2nd step

The 2nd step is a step of filling the sevoflurane storage container, in which sevoflurane and its analogs (polyether 1 and polyether 2) become substantially not detected as a result of the 1 st step, with fresh sevoflurane. A pharmaceutical product of sevoflurane filled into a storage container can be produced by conducting the 2nd step, in addition to the 1st step.

Sevoflurane is in the form of liquid with a boiling point of 58.6°C. It may be filled into a container as in the case of a common liquid substance. It is preferable for sevoflurane to contain water at 206 ppm to 1400 ppm as disclosed in Patent Literature 5 so that stability of sevoflurane can be further improved.

It is preferable to conduct the 2nd step by, for example, replacing the air inside of a storage container by an inert gas (e.g., dry nitrogen) at atmospheric pressure in advance and filling the storage container with a predetermined volume of sevoflurane for a new lot. This operation allows the inert gas present in the container to be discharged from a depressurization port. Usually, after being filled into the container, sevoflurane gradually forms vapor in a gas phase, thereby causing the inside of the container to be slightly pressurized as stated above.

Another option is to evacuate the air from a storage container in advance and introduce sevoflurane thereinto, which also falls within the scope of the present invention.

The thus obtained pharmaceutical product produced can be subjected to gas chromatographic analysis by collecting a small amount of sevoflurane filled into the container (i.e., "product analysis"). There are substantially no impurities in the sevoflurane storage container after the 1st step (steps A to C or a to c). After the completion of the 2nd step, sevoflurane filled into the container is analyzed again, thereby making it possible to confirm whether washing has been completed for each batch and ensure quality assurance.

In fact, after washing of the container using a "solvent substantially consisting of water" according to the present invention, sevoflurane analogs (polyether 1 and polyether 2) were not detected with significance during "product analysis" even after the container was used for many lots. This demonstrates effectiveness of the present invention.

### [3] Regarding the 3rd step

The 3rd step is a step of continuously storing the sevoflurane storage container filled with sevoflurane as described in the 2nd step. By carrying out the 1st step, the 2nd step, and the 3rd step in such order, sevoflurane for a new lot can be preferably stored.

As sevoflurane filling the storage container after the 1st and 2nd steps has high stability, the storage temperature is not particularly limited. However, in consideration of the use of sevoflurane as a medicine, it is preferable to store sevoflurane at a low temperature below the boiling point (58.6°C). Sevoflurane is stored at preferably 0°C to 35°C and more preferably at a temperature close to room temperature (e.g., 10°C to 30°C).

### EXAMPLES

The present invention will be described more in detail below with reference to the following Examples. However, the present invention is not limited thereto. It is to be noted that "liquid substantially consisting of water" (simply referred to as "water" in some cases) was used as a washing agent in the following Examples, Comparative Examples, and Reference Examples. Specifically, the liquid is "ion-exchange water having an electric conductivity of 1 µS/cm or less."

### [Example 1]

In Example 1, sevoflurane was treated under conditions more stringent than actual sevoflurane storage conditions so that "polyether" was forcibly generated. Specifically, according to the disclosure of Patent Literature 5, the water content of sevoflurane was adjusted to be lower than that of sevoflurane obtained as a final product (6 ppm). Further, sevoflurane was heated to 50°C under the presence of oxidized aluminium (alumina) serving as Lewis acid (i.e., a substance that is considered to catalyze sevoflurane degradation in Patent Literature 5). As a result, a portion of sevoflurane was degraded into polyether, resulting in generation of "sevoflurane containing polyether" in the system. It was attempted to wash the obtained "sevoflurane containing polyether" with water.

### (1-1) Drying of sevoflurane (reduction of water content)

Sevoflurane (used as a pharmaceutical product) (100 cm³) was placed in a erlenmeyer flask and cooled to 10°C in a refrigerator. Then, synthetic zeolite was introduced into the erlenmeyer flask so that the weight ratio of synthetic zeolite : sevoflurane became 1:4. Next, the erlenmeyer flask was allowed to stand still in a refrigerator at 10°C for 2 hours and 30 minutes for drying of sevoflurane (reduction of water content). Thereafter, the moisture of sevoflurane was determined using a Karl Fischer moisture meter and it was found to be 6 ppm.

### (1-2) Degradation of sevoflurane (severe testing)

Alumina (50 mg) was placed in a 50-cm³ brown bottle with a narrow mouth. Sevoflurane having a moisture content of 6 ppm prepared in (1-1) (20 cm³, 29.9 g) was added thereto.

Next, the cap of the brown bottle with a narrow mouth was closed and the bottle was heated in a thermostatic bath at 50°C for 64 hours. After heating for 64 hours, the bottle was cooled in a refrigerator (10°C) for 1 hour.

### (1-3) Removal of hydrogen fluoride

As the sevoflurane obtained as a result of severe testing in (1-2) contained hydrogen fluoride (HF), water washing (back extraction of HF into an aqueous phase) was conducted for the ease of handling. Specifically, the sevoflurane subjected to severe testing and water (15 cm³) were introduced into a separatory funnel made of tetrafluoroethylene, stirred for 1 minute, and allowed to stand still for 1 minute, followed by liquid separation. Then, the aqueous phase was collected and pH was checked using a pH test paper.

The above operations were repeated 3 times. The results for the first, second, and third pH tests of the aqueous phase were pH = 1, pH = 4, and pH = 6, respectively. It was judged that hydrogen fluoride was removed from the organic phase as a result of the third washing.

Meanwhile, after the third water washing, the gas chromatographic composition of the organic phase was detected as follows: sevoflurane: about 87%; polyether 1: 8.4% (84000 ppm); and polyether 2: 4.0% (40000 ppm). In addition, 190 ppm of hexafluoroisopropyl alcohol (HFIP) was detected.

Here, although the detailed mechanism of the reaction of converting sevoflurane into "polyether 1" and "polyether 2" is unknown, the reaction would be a series of reactions involving Lewis acid as disclosed in Patent Literature 5 (see column 4).

Patent Literature 5 discloses that HFIP (hexafluoroisopropyl alcohol) is also generated in the degradation reaction. As an aside, HFIP is a water-soluble substance and is also an "amphipathic substance" having lipophilicity. It is therefore considered that a large portion of HFIP could be released into the aqueous phase by repeating water washing (back extraction) 3 times in the above manner, which allowed only a portion of HFIP to remain in the organic phase and resulted in the above HFIP content ("190 ppm").

Meanwhile, the content of either polyether 1 or 2 in the organic phase was more than 100 times that of HFIP. This suggests that both polyether 1 and polyether 2 are characterized by very high lipophilicity and very low affinity for water as described herein (meaning that it is impossible to treat polyether 1 and polyether 2 by back extraction with water).

### (1-4 Container washing with water)

(a) The "sevoflurane containing polyether 1 and polyether 2" prepared in (1-3) above was collected (5 cm³, about 10 g) and placed in an autoclave made of stainless steel (SUS304) (inner volume: 500 cm³). After having been hermetically closed, the autoclave was shaken for 10 minutes so that the inner wall of the autoclave was totally covered with the liquid. Then, the autoclave was allowed to stand still at room temperature for 24 hours (this operation corresponds to "step A"). Note that the autoclave used herein has a washing liquid inlet port and an air vent port at its top and a water discharge port at its bottom.
(b) The water discharge port of the autoclave was opened to drain the liquid inside of the autoclave. Then, the port was kept open for 1 minute for liquid draining.
(c) The water discharge port was closed and then 12.5 cm³ of water heated to 80°C was added. Thereafter, the storage container was hermetically sealed. The autoclave was shaken for 10 minutes so that the added water was brought into contact with the entire inner wall of the autoclave ("the first water washing ").
   Next, water discharge port of the autoclave was opened to drain the liquid inside of the autoclave. Then, the port was kept open for 1 minute for liquid draining.
   As described above, the total amount of drained liquid was collected, ice-cooled, mixed with 0.5 cm³ of diethyl ether, and shaken well. Accordingly, the lipophilic component was extracted. Then, the diethyl ether solution (referred to as "the composition of wash liquid of the first water washing") was analyzed by gas chromatography (FID). As a result, the concentrations of "polyether 1" and "polyether 2" based on the total peak area including the peak of diethyl ether were 65 ppm and 76 ppm, respectively (although the peak of sevoflurane was also detected, it was not quantitatively determined because most of sevoflurane had evaporated during washing with water at 80°C).
(d) The autoclave treated in (c) above was further subjected to "the second water washing" with the use of 12.5 cm³ of water (80°C) in the manner described in (c). After extraction into diethyl ether, the composition was determined by gas chromatography (FID). Accordingly, "the composition of wash liquid of the second water washing" was found to comprise "polyether 1" at 8 ppm and "polyether 2" at 13 ppm.
(e) The autoclave treated in (d) above was further subjected to "the second water washing" with the use of 12.5 cm³ of water (80°C) in the manner described in (d). After extraction into diethyl ether, the composition was determined by gas chromatography (FID). Accordingly, "the composition of wash liquid of the third water washing" was found to comprise "polyether 1" at 1 ppm and "polyether 2" at 2 ppm.

Subsequently, "the fourth water washing (80°C)," "the fifth water washing (80°C)," and "the sixth water washing (80°C)" were conducted in the manner described above. The wash liquid composition was determined after each washing. Both "polyether 1" and "polyether 2" were not detected (less than 1 ppm). That is to say, it was revealed that it is possible to effectively discharge "polyether 1 and polyether 2" together with flowing water outside of the system by repeatedly conducting water washing while preventing them from adhering to the inner surface of the storage container.

### (1-5 Container washing with methanol)

For confirmation, 2.5 cm³ of ice-cooled methanol was introduced into the autoclave after (1-4) above, and washing was conducted in the manner described above. The methanol wash liquid was drained from the drainpipe and the methanol was subjected to gas chromatographic analysis. Accordingly, none of "sevoflurane," "polyether 1," and "polyether 2" was detected.

That is, it was confirmed that it was possible to wash the sevoflurane storage container to a sufficient extent by conducting "water washing" in (1-4) without conducting "methanol washing" in (1-5).

As stated above, polyether 1 and polyether 2 were forcibly generated by treating sevoflurane under conditions more stringent than actual conditions, and the obtained "sevoflurane containing polyether 1 and polyether 2" was subjected to water washing in Example 1. As a result, although "polyether 1 and polyether 2" were detected in "wash liquid of the third water washing," the contents of "polyether 1 and polyether 2" decreased as washing was repeated. After "the fourth water washing," "polyether 1 and polyether 2" were not detected. That is, the results support that even if "polyether 1 and polyether 2" having poor affinity for water are generated, they can be reduced to a level that is pharmaceutically safe with the use of "liquid substantially consisting of water," which was unpredictable in the past.

### [Example 2]

### (Step A)

A predetermined volume (500 dm³) of a sevoflurane product filled into a stainless-steel (SUS304) storage container (inner volume: 500 dm³) (i.e., a sevoflurane product filled into the container for a previous lot) was pressurized using dry nitrogen and collected via a dip tube. When it was found that no more liquid could be collected, both the nitrogen gas inlet port and the dip tube inlet were immediately closed.

This resulted in a state in which sevoflurane vapor was present substantially at its saturated vapor pressure in the storage container. This state was maintained at room temperature for one week.

### (Step B)

A "horizontally rotatable spray nozzle <a>" was attached to the "storage container in which at least sevoflurane vapor was present" treated in step A as shown in Figure 2, followed by water injection. The width of a water injection slit of the spray nozzle <a> (see Figure 3) was determined to be about 0.6 mm. The water pressure of water injected by a spray nozzle was set to 0.4 MPa (absolute pressure) and the water flow rate was set to 13.6 dm³/minute. The spray nozzle <a> is horizontally rotatable when water pressure is applied. Thus, its rotational rate was 30 to 40 rpm (0.50 to 0.67 rotations/second) under the above conditions. The water temperature (i.e., water temperature in a water reservoir attached to the spray nozzle <a> at the position adjacent to the storage container) was set to 80°C.

A drainpipe <b> provided to the lower part of the storage container was kept open from the beginning of step B so that water injection and water discharge were carried out therethrough. At first, water injection was carried out for 3 minutes and then the storage container was allowed to stand still for 60 seconds. Next, an operation of "washing for 15 seconds and allowing the storage container to stand still for 30 seconds" was repeated 10 times. As a result of the operation, water injection was carried out for 330 seconds in total. The total volume of water injected during 330 seconds was 75 dm³.

### (Step C)

After step B, the storage container was subjected to step C in the embodiment shown in Figure 3. Specifically, step C was carried out in a manner such that a drying gas was introduced from the upper part and discharged from the drainpipe provided to the lower part.

The drying gas used herein was "dry air having a dew point of -40°C or less (-40°C to -50°C)." The drying gas was heated with a heater so as to adjust the temperature of the drying gas (immediately before being introduced into the storage container) to 60°C to 80°C. The flow rate of the drying gas to be introduced was set to 170 dm³/minute. The drying gas was continuously introduced into/discharged from the storage container for 2 hours and 40 minutes.

After the elapse of 2 hours and 40 minutes, introduction of the drying gas was discontinued and both the inlet and the drainpipe were closed. Then, dry air was introduced to increase the pressure inside of the storage container to 0.07 MPa. When the pressurized gas inside of the storage was discharged (purged), the dew point of the gas was measured and found to be -30°C, confirming that water used as washing liquid had been completely removed.

### (Refilling and analysis)

After the completion of dew point measurement, the storage container was filled with sevoflurane for a new lot. Sevoflurane was collected from the storage container after filling, followed by analysis. It was found that the collected sevoflurane satisfied all test standards.

### [Example 3]

The operations of the experiment in Example 2 except for the operation described in "Refilling and analysis" were repeated using the same devices (the 500-dm³ sevoflurane storage container (SUS304), the rotatable spray nozzle, etc.).

After the completion of the operations corresponding to steps A to C, the container was cooled to room temperature. Then, the cap of the container was opened and 1.5 dm³ of methanol (room temperature) was directly applied to the entire inner wall of the container, instead of carrying out "refilling and analysis" described in Example 2. Subsequently, methanol wash liquid that was being drained from the drainpipe was collected and analyzed.

As a result, the concentrations of "polyether 1 and polyether 2" were below the detection limit (1 ppm). That is, when additional washing with methanol was conducted after the operations corresponding to steps A to C for confirmation, "polyether 1 and polyether 2" were not detected. Thus, effectiveness of washing in steps A to C was emphasized with certainty.

### [Example 4]

After the completion of the washing operations (steps A to C) in Example 2, the sevoflurane storage container was filled with sevoflurane (500 dm³) for a new lot. Then, after the completion of a step of consuming (collecting) sevoflurane, the storage container was subjected to steps A to C by the means used in Example 2. A series of these operations was repeated using the same container for 20 lots.

During the repetition of the above operations, when the storage container was filled with sevoflurane for a new lot after the completion of the washing step, "polyether 1 and polyether 2" were never detected as analogs as a result of product analysis after filling. It was found that all sevoflurane quality standards were satisfied. That is, it was demonstrated that quality assurance of sevoflurane can be achieved by the washing method of the present invention with certainty.

### [Reference Example 1]

A 1-dm³ autoclave (SUS304) used as a sevoflurane storage container was filled with dry nitrogen. Liquid sevoflurane (i.e., a sevoflurane product containing 400 ppm of water) (30 g) was added thereto. The autoclave was hermetically closed and stored at room temperature for 1 month. Then, moisture content was measured using a Karl Fischer moisture meter. The moisture content was found to be 360 ppm. The liquid sevoflurane was stored for another month in the autoclave in the same manner. Then, the moisture content was measured again. The moisture content was found to be 350 ppm.

As described above, when a small amount of sevoflurane is stored with a large amount of a drying gas in a large-capacity storage container, the moisture content in a liquid phase may decrease in a time-dependent manner, although the reason is unclear. In fact, the water content usually does not significantly decrease as in the case of "Example 1." However, if a small amount of sevoflurane remains at the bottom of a storage container after the completion of a step of consuming (collecting) sevoflurane, such sevoflurane might behave differently from usual "sevoflurane" filled into a storage container. In view of this, the data obtained above suggest that it is highly required to conduct washing of the storage container in a careful manner in order to achieve quality assurance of sevoflurane with certainty.

### [Reference Example 2]

"Sevoflurane containing polyether 1 and polyether 2" was prepared in accordance with the procedures and amounts described in (1-1) to (1-3) of "Example 1." Next, " rinsing with sevoflurane" was conducted instead of (1-4).

Specifically, the procedures of (a) and (b) in (1-4) of "Example 1" were performed as in Example 1. Then, washing was carried out 3 times using 12.5 cm³ of a "sevoflurane product" (room temperature) instead of using 12.5 cm³ of water in (c) in (1-4) of "Example 1." The resulting sevoflurane wash liquid was drained from the drainpipe and the sevoflurane wash liquid was subjected to gas chromatographic analysis. As a result, the concentrations of "polyether 1" and "polyether 2" in sevoflurane drained from the drainpipe were 4 ppm and 5 ppm, respectively, for the first measurement. The concentration of "polyether 2" in sevoflurane drained for the second measurement was 1 ppm while no "polyether 1" was detected. Neither "polyether 1" nor "polyether 2" was detected from sevoflurane drained for the third measurement.

As stated above, it was confirmed that "polyether 1 and polyether 2" can be sufficiently washed away using expensive sevoflurane as a washing liquid due to lipophilicity of sevoflurane.

### [Reference Example 3]

The procedures of Example 1 were conducted in the same manner except that the procedures of (a) and (b) in (1-4) were changed as described below.

Specifically, "sevoflurane containing polyether" was introduced into a 500-cm³ autoclave and brought into contact well with the inner wall of the autoclave. Then, the water discharge port of the autoclave was immediately opened to drain the liquid inside thereof without conducting the procedure of "allowing the autoclave to stand still for 24 hours" (a) in (1-4) (step A). Thereafter, the cover of the autoclave was kept open and left at room temperature for 3 days. Accordingly, sevoflurane in the sample evaporated, and therefore, the inner wall of the container was dried.

Subsequently, the first water washing was carried out in the manner described above, followed by ice cooling and extraction into diethyl ether. As a result, the concentrations of "polyether 1" and "polyether 2" were 7 ppm and 12 ppm, respectively. As a result of the second water washing, the concentrations of "polyether 1" and "polyether 2" were 5 ppm and 9 ppm, respectively. As a result of the third water washing, the concentrations of "polyether 1" and "polyether 2" were 3 ppm and 7 ppm, respectively.

Once the inside of the storage container was dried without carrying out step A as described above, it became sometimes difficult to remove polyether even by conducting water washing.

### [Comparative Example 1]

"Sevoflurane containing polyether 1 and polyether 2" was prepared in accordance with the procedures and amounts described in (1-1) to (1-3) of "Example 1." Next, "sevoflurane containing polyether 1 and polyether 2" was brought into contact well with the inner wall of the container in the operation corresponding to (1-4) of "Example 1." Then, "container washing with acetone" was carried out instead of "water washing."

Specifically, the procedures of (a) and (b) in (1-4) of "Example 1" were performed as in Example 1. Then, washing was carried out 3 times using 12.5 cm³ of acetone (room temperature) instead of using 12.5 cm³ of water in (c) in (1-4) of "Example 1." The resulting acetone wash liquid was drained from the drainpipe and the acetone wash liquid was subjected to gas chromatographic analysis. As a result, the concentrations of "polyether 1" and "polyether 2" in acetone drained from the drainpipe were 3 ppm and 2 ppm, respectively, for the second measurement. Neither "polyether 1" nor "polyether 2" was detected from acetone drained for the third measurement.

Subsequently, dry air at 60°C to 80°C was allowed to circulate in the storage container for 60 minutes. Then, 3 cm³ of a sevoflurane product was introduced again into the storage container and brought into contact well with the inner surface of the storage container. The sevoflurane was subjected to gas chromatographic analysis.

As a result, the peak of a small amount of acetone was detected while "polyether 1 and polyether 2" were not detected.

When acetone is used as a washing liquid as described above, it would be unexpectedly difficult to remove acetone, although it would be possible to remove "polyether 1 and polyether 2." This suggests that washing with acetone could not always be efficient washing means.

### INDUSTRIAL APPLICABILITY

According to the present invention, it is possible to effectively wash a used "sevoflurane storage container" with an inexpensive liquid containing water as a major component. Specifically, washing can be carried out to such an extent that substantially none of sevoflurane and its analogs (polyether 1 and polyether 2) can be detected inside of a storage container.

Moreover, according to the present invention, it is possible to separately carry out the step of washing the storage container and the subsequent step of filling the storage container with sevoflurane, thereby allowing the degree of freedom of each step to increase.

## Claims

1. A method for washing a sevoflurane storage container, wherein
the storage container is a sevoflurane storage container, which is repeatedly used for a series of lots, the storage container having been subjected to a step of consuming at least a portion of sevoflurane filled into the storage container for a previous lot,
the method comprising the steps of:
creating a state in which at least sevoflurane vapor is present in the storage container (step A);
bringing a liquid containing water as a major component into contact with the inner wall of the sevoflurane storage container in the state in which sevoflurane vapor is present in the storage container after step A and draining the liquid outside of the storage container while the liquid remains liquid (step B); and
introducing a drying gas into the storage container so as to drain the liquid remaining on the inner wall of the storage container together with the drying gas outside of the storage container after step B (step C).

2. The washing method according to claim 1, wherein the state in which at least sevoflurane vapor is present in the storage container in step A is a state that is achieved by consuming substantially all liquid sevoflurane filled into the storage container in the step of consuming sevoflurane filled into the storage container for a previous lot and then hermetically sealing the storage container while allowing at least a portion of sevoflurane vapor to remain in a gas phase in the storage container.

3. The washing method according to claim 1, wherein the state in which at least sevoflurane vapor is present in the storage container in step A is a state that is achieved by consuming a portion of liquid sevoflurane filled into the storage container in the step of consuming sevoflurane filled into the storage container for a previous lot and then hermetically sealing the storage container.

4. The washing method according to any one of claims 1 to 3, wherein the liquid containing water as a major component is a liquid substantially consisting of water.

5. The washing method according to any one of claims 1 to 4, wherein in order to bring a liquid containing water as a major component into contact with the inner wall of the sevoflurane storage container in step B, the liquid is directly injected to the inner wall of the storage container using liquid injection means.

6. The washing method according to claim 5, wherein the temperature of the liquid containing water as a major component is 60°C to 90°C upon injection.

7. The washing method according to any one of claims 1 to 6, wherein a part of or all of the inner wall of the storage container is made of at least one material selected from the group consisting of stainless steel, resin lining, and glass.

8. The washing method according to claim 7, wherein a part of or all of the inner wall of the storage container is made of stainless steel.

9. The washing method according to any one of claims 1 to 8, wherein the drying gas in step C is dry air at 30°C to 150°C.

10. The washing method according to any one of claims 1 to 9, wherein step C includes confirming that the drying gas discharged from the storage container has a dew point at or lower than a predetermined temperature.

11. A method for washing a sevoflurane storage container, wherein
the storage container is a sevoflurane storage container, which is repeatedly used for a series of lots, the storage container having been subjected to a step of consuming at least a portion of sevoflurane filled into the storage container for a previous lot,
the method comprising the steps of:
creating a state in which at least sevoflurane vapor is present in the storage container by hermetically sealing the storage container while allowing at least a portion of sevoflurane vapor to remain in a gas phase in the storage container after consuming sevoflurane in the step of consuming sevoflurane filled into the storage container for a previous lot (step a);
directly spraying a liquid substantially consisting of water the inner wall of the storage container using a spray nozzle so as to bring the liquid into contact with the inner wall in a state in which sevoflurane vapor is present in the storage container after step a and then draining the liquid outside of the storage container while the liquid remains liquid (step b); and
introducing a drying gas into the storage container so as to drain the liquid remaining on the inner wall of the storage container together with the drying gas outside of the storage container after step b and confirming whether the dew point of the drying gas discharged from the storage container is at or lower than a predetermined temperature (step c),
wherein as a result of implementation of steps a to c, sevoflurane and its analog compounds (polyether 1 and polyether 2) expressed by the following formula are substantially not detected in the storage container:
(CF₃)₂CHO-CH₂-O-CH(CF₃)₂ <Polyether 1>
(CF₃)₂CHO-CH₂-O-CH₂-O-CH(CF₃)₂ <Polyether 2>

12. The washing method according to claim 11, wherein a part of or all of the inner wall of the storage container is made of stainless steel, and the temperature of the liquid substantially consisting of water is 60°C to 90°C.

13. The washing method according to any one of claims 1 to 12, wherein step B or step b includes a step of washing the storage container using purified sevoflurane.

14. A method for producing a pharmaceutical product, which is sevoflurane filled into a storage container, the method comprising filling a storage container washed by the method according to any one of claims 1 to 13 with sevoflurane for a new lot.

15. The washing method according to claim 14, wherein the liquid composition of sevoflurane filled into a storage container for a new lot is assayed by gas chromatography.

16. A pharmaceutical product of sevoflurane filled into a storage container, which is produced by the method according to claim 14 or 15.

17. A method for storing sevoflurane, comprising the following 1st to 3rd steps:
the 1st step of washing a sevoflurane storage container by the method according to any one of claims 1 to 13;
the 2nd step of filling the storage container with sevoflurane for a new lot after washing; and
the 3rd step of storing the sevoflurane storage container after filling.

## Amended claims

### Amended claims in accordance with Rule 137(2) EPC.

**1.** A method for washing a sevoflurane storage container, wherein
the storage container is a sevoflurane storage container, which is repeatedly used for a series of lots, the storage container having been subjected to a step of consuming at least a portion of sevoflurane filled into the storage container for a previous lot,
the method comprising the steps of:
step A - creating a state in which at least sevoflurane vapor is present in the storage container by hermetically sealing the storage container while allowing at least a portion of sevoflurane vapor to remain in a gas phase in the storage container after consuming sevoflurane in the step of consuming sevoflurane filled into the storage container for a previous lot;
step B - bringing a liquid composed of a substance comprising water molecules that account for 99.9% by mass or more of the substance into contact with the inner wall of the sevoflurane storage container in the state in which sevoflurane vapor is present in the storage container after step A and draining the liquid outside of the storage container while the liquid remains liquid; and
step C - introducing a drying gas into the storage container so as to drain the liquid remaining on the inner wall of the storage container together with the drying gas outside of the storage container after step B.

**2.** The washing method according to claim 1, wherein the liquid composed of a substance comprising water molecules is a liquid consisting of water.

**3.** The washing method according to either claim 1 or 2, wherein in order to bring a liquid composed of a substance comprising water molecules into contact with the inner wall of the sevoflurane storage container in step B, the liquid is directly injected to the inner wall of the storage container using liquid injection means.

**4.** The washing method according to claim 3, wherein the temperature of the liquid composed of a substance comprising water molecules is 60°C to 90°C upon injection.

**5.** The washing method according to any one of claims 1 to 4, wherein a part of or all of the inner wall of the storage container is made of at least one material selected from the group consisting of stainless steel, resin lining, and glass.

**6.** The washing method according to claim 5, wherein a part of or all of the inner wall of the storage container is made of stainless steel.

**7.** The washing method according to any one of claims 1 to 6, wherein the drying gas in step C is dry air at 30°C to 150°C.

**8.** The washing method according to any one of claims 1 to 7, wherein step C includes confirming that the drying gas discharged from the storage container has a dew point at or lower than -20°C when using a drying gas having a dew point of -30°C or less or -30°C when using a drying gas having a dew point of -40°C or less.

**9.** A method for washing a sevoflurane storage container according to claim 1, wherein
the storage container is a sevoflurane storage container, which is repeatedly used for a series of lots, the storage container having been subjected to a step of consuming at least a portion of sevoflurane filled into the storage container for a previous lot,
the method comprising the steps of:
step a - creating a state in which at least sevoflurane vapor is present in the storage container by hermetically sealing the storage container while allowing at least a portion of sevoflurane vapor to remain in a gas phase in the storage container after consuming sevoflurane in the step of consuming sevoflurane filled into the storage container for a previous lot;
step b - directly spraying a liquid composed of a substance comprising water molecules that account for 99.9% by mass or more of the substance onto inner wall of the storage container using a spray nozzle so as to bring the liquid into contact with the inner wall in a state in which sevoflurane vapor is present in the storage container after step a and then draining the liquid outside of the storage container while the liquid remains liquid; and
step c - introducing a drying gas into the storage container so as to drain the liquid remaining on the inner wall of the storage container together with the drying gas outside of the storage container after step b and confirming whether the dew point of the drying gas discharged from the storage container is at or lower than -20°C when using a drying gas having a dew point of -30°C or less or -30°C when using a drying gas having a dew point of -40°C or less,
wherein as a result of implementation of steps a to c, sevoflurane and its analog compounds polyether 1 and polyether 2 expressed by the following formula are substantially not detected in the storage container:
(CF₃)₂CHO-CH₂-O-CH(CF₃)₂ < Polyether 1 >
(CF₃)₂CHO-CH₂-O-CH₂-O-CH(CF₃)₂ < Polyether 2 >

**10.** The washing method according to claim 9, wherein a part of or all of the inner wall of the storage container is made of stainless steel, and the temperature of the liquid composed of a substance comprising water molecules is 60°C to 90°C.

**11.** The washing method according to any one of claims 1 to 10, wherein step B or step b includes a step of washing the storage container using purified sevoflurane.

**12.** A method for producing a pharmaceutical product, which is sevoflurane filled into a storage container, the method comprising filling a storage container washed by the method according to any one of claims 1 to 11 with sevoflurane for a new lot.

**13.** The washing method according to claim 12, wherein the liquid composition of sevoflurane filled into a storage container for a new lot is assayed by gas chromatography.

**14.** A method for storing sevoflurane, comprising the following 1st to 3rd steps:
the 1st step of washing a sevoflurane storage container by the method according to any one of claims 1 to 11;
the 2nd step of filling the storage container with sevoflurane for a new lot after washing; and
the 3rd step of storing the sevoflurane storage container after filling.

**1.** A method for washing a sevoflurane storage container, wherein
the storage container is a sevoflurane storage container, which is repeatedly used for a series of lots, the storage container having been subjected to a step of consuming at least a portion of sevoflurane filled into the storage container for a previous lot,
the method comprising the steps of:
step A - creating a state in which at least sevoflurane vapor is present in the storage container by hermetically sealing the storage container while allowing at least a portion of sevoflurane vapor to remain in a gas phase in the storage container after consuming sevoflurane in the step of consuming sevoflurane filled into the storage container for a previous lot;
step B - bringing a liquid composed of a substance comprising water molecules that account for 99.9% by mass or more of the substance into contact with the inner wall of the sevoflurane storage container in the state in which sevoflurane vapor is present in the storage container after step A and draining the liquid outside of the storage container while the liquid remains liquid, wherein the temperature of the liquid composed of a substance comprising water molecules is 60°C to 90°C; and
step C - introducing a drying gas into the storage container so as to drain the liquid remaining on the inner wall of the storage container together with the drying gas outside of the storage container after step B.

**2.** The washing method according to claim 1, wherein the liquid composed of a substance comprising water molecules is a liquid consisting of water.

**3.** The washing method according to either claim 1 or 2, wherein in order to bring a liquid composed of a substance comprising water molecules into contact with the inner wall of the sevoflurane storage container in step B, the liquid is directly injected to the inner wall of the storage container using liquid injection means.

**4.** The washing method according to any one of claims 1 to 3, wherein a part of or all of the inner wall of the storage container is made of at least one material selected from the group consisting of stainless steel, resin lining, and glass.

**5.** The washing method according to claim 4, wherein a part of or all of the inner wall of the storage container is made of stainless steel.

**6.** The washing method according to any one of claims 1 to 5, wherein the drying gas in step C is dry air at 30°C to 150°C.

**7.** The washing method according to any one of claims 1 to 6, wherein step C includes confirming that the drying gas discharged from the storage container has a dew point at or lower than -20°C when using a drying gas having a dew point of -30°C or less or -30°C when using a drying gas having a dew point of -40°C or less.

**8.** A method for washing a sevoflurane storage container according to claim 1, wherein
the storage container is a sevoflurane storage container, which is repeatedly used for a series of lots, the storage container having been subjected to a step of consuming at least a portion of sevoflurane filled into the storage container for a previous lot,
the method comprising the steps of:
step a - creating a state in which at least sevoflurane vapor is present in the storage container by hermetically sealing the storage container while allowing at least a portion of sevoflurane vapor to remain in a gas phase in the storage container after consuming sevoflurane in the step of consuming sevoflurane filled into the storage container for a previous lot;
step b - directly spraying a liquid composed of a substance comprising water molecules that account for 99.9% by mass or more of the substance onto inner wall of the storage container using a spray nozzle so as to bring the liquid into contact with the inner wall in a state in which sevoflurane vapor is present in the storage container after step a and then draining the liquid outside of the storage container while the liquid remains liquid, wherein the temperature of the liquid composed of a substance comprising water molecules is 60°C to 90°C; and
step c - introducing a drying gas into the storage container so as to drain the liquid remaining on the inner wall of the storage container together with the drying gas outside of the storage container after step b and confirming whether the dew point of the drying gas discharged from the storage container is at or lower than -20°C when using a drying gas having a dew point of -30°C or less or -30°C when using a drying gas having a dew point of -40°C or less,
wherein as a result of implementation of steps a to c, sevoflurane and its analog compounds polyether 1 and polyether 2 expressed by the following formula are substantially not detected in the storage container:
(CF₃)₂CHO-CH₂-O-CH(CF₃)₂ < Polyether 1 >
(CF₃)₂CHO-CH₂-O-CH₂-O-CH(CF₃)₂ < Polyether 2 >

**9.** The washing method according to claim 9, wherein a part of or all of the inner wall of the storage container is made of stainless steel.

**10.** The washing method according to any one of claims 1 to 9, wherein step B or step b includes a step of washing the storage container using purified sevoflurane.

**11.** A method for producing a pharmaceutical product, which is sevoflurane filled into a storage container, the method comprising filling a storage container washed by the method according to any one of claims 1 to 10 with sevoflurane for a new lot.

**12.** The washing method according to claim 11, wherein the liquid composition of sevoflurane filled into a storage container for a new lot is assayed by gas chromatography.

**13.** A method for storing sevoflurane, comprising the following 1st to 3rd steps:
the 1st step of washing a sevoflurane storage container by the method according to any one of claims 1 to 10;
the 2nd step of filling the storage container with sevoflurane for a new lot after washing; and
the 3rd step of storing the sevoflurane storage container after filling.
